# EUROPEAN PATENT SPECIFICATION

(11) **EP 1 764 419 B1**
(45) Date of publication and mention of the grant of the patent: **25.04.2012**
(21) Application number: 06254777.3
(22) Date of filing: 14.09.2006
(51) Int. Cl.: C12Q 1/68

(54) **Detecting gene methylation for diagnosis of a proliferative disorder**
Verfahren zur Diagnose proliferativer Erkrankungen durch Detektion von Genmethylierung
Procédé pour détecter la méthylation des gènes pour diagnostiquer une maladie proliferative

(30) Priority: 15.09.2005 US 717790 P
(43) Date of publication of application: 21.03.2007
(73) Proprietor: Veridex, LLC, Warren, NJ 07059 (US)
(72) Inventor: Vener, Tatiana, Warren, NJ 07059 (US); Mehrotra, Jyoti, Warren, NJ 07059 (US); Varde, Shobha, Warren NJ 07059 (US); Mazumder, Abhijit, Warren NJ 07059 (US); Baden, Jon, Warren NJ 07059 (US); Backus, John, Raritan, NJ 08869 (US)
(74) Representative: Mercer, Christopher Paul

(56) References cited:
- WO-A-99/55905
- WO-A-03/044232
- WO-A-2004/067777
- WO-A2-2004/087957
- WO-A2-2004/113567
- WO-A2-2005/001140
- WO-A2-2005/042713
- DATABASE Geneseq [Online] 9 September 2004 (2004-09-09), "PCR primer amplifies promoter CpG islands of cancer related genes Seq152." XP002418712 retrieved from EBI accession no. GSN:ADQ77470 Database accession no. ADQ77470
- DATABASE Geneseq [Online] 21 October 2004 (2004-10-21), "GSTP1 probe, oligo--probe, SEQ ID 17." XP002418713 retrieved from EBI accession no. GSN:ADR11806 Database accession no. ADR11806 & WO 2004/065625 A (HUMAN GENETIC SIGNATURES PTY L [AU]; MILLAR DOUGLAS SPENCER [AU]; MELK) 5 August 2004 (2004-08-05)
- CHU ET AL: "The Use Of Real-Time Quantitative Polymerase Chain Reaction To Detect Hypermethylation Of The CpG Islands In The Promoter Region Flanking The GSTP1 Gene To Diagnose Prostate Carcinoma" JOURNAL OF UROLOGY, BALTIMORE, MD, US, vol. 167, no. 4, April 2002 (2002-04), pages 1854-1858, XP005543746 ISSN: 0022-5347

## Description

### BACKGROUND OF THE INVENTION

This invention relates to the interrogation of methylated genes in diagnostic and prognostic assays for cellular proliferative disorders such as cancer. The genes involved include the glutathione-S-transferase (GSTP1) gene or portions of

In higher order eukaryotes DNA is methylated only at cytosines located 5' to guanosine in the CpG dinucleotide. This modification has important regulatory effects on gene expression, especially when it involves CpG rich areas (CpG islands) located in gene promoter regions. Abberant methylation of normally unmethylated CpG islands is a frequent event in immortalized and transformed cells and has been associated with transcriptional inactivation of certain tumor suppressor genes or genes otherwise associated with the amelioration of certain human cancers.

Glutathione S-transferases (GSTs) catalyze intracellular detoxification reactions, including the inactivation of electrophilic carcinogens, by conjugating chemically-reactive electrophiles to glutathione (C. B. Pickett, et al., Annu. Rev. Biochem., 58:743, 1989; B. Coles, et al., CRC Crit. Rev. Biochem. Mol. Biol., 25:47, 1990; T. H. Rushmore, et al., J. Biol. Chem. 268:11475, 1993). Human GSTs, encoded by several different genes at different loci, have been classified into four families referred to as alpha, mu, pi, and theta (B. Mannervik, et al., Biochem. J., 282:305, 1992). Many human cancers such as prostate and hepatic cancers exhibit decreased GSTP1 expression relative to their tissues of origin.

WO2004087957 and WO2004067777 disclose methods for predicting the course of a proliferative disorder wherein the methylation status of the marker GSTP1 is determined using a reagent consisting of the sequence gccccaatactaaatcacgacg. WO2004113567 relates to methods wherein part of the GSTpI is amplified in order to assess its methylation status.

In the case of prostate cancer, in particular, designing methylation specific PCR ("MSP") primers and probes that discriminate between benign prostatic hyperplasia and prostate adenocarcinoma has proven to be complex. The best published primer/probe sets typically display up to about 75% sensitivity if one adjusts the assays to be 100% specific and less than 60% specific if one adjusts the assays to be 100% sensitive. A more robust and consistent assay might be obtained with new primer and probe combinations. The present invention fulfills such a need.

### SUMMARY OF THE INVENTION

In one aspect, the invention provides a method of diagnosing the presence or predicting the course of a proliferative disorder comprising determining the methylation status of a Marker for GSTP1 in a biological sample with the use of a nucleic acid probe and primers encoded by Seq. ID NOs 23-25.

In one aspect of the disclosure, a method for detecting a cell proliferative disorder in prostatic tissue or other sample of a subject involves contacting a cellular component with a reagent useful in the amplification and detection of hypermethylated regions of certain genes.

In another aspect of the disclosure, at least one such hypermethylated gene is GSTP1 I which can be assayed in combination with one or more other genes.

In another aspect of the disclosure, a nucleic acid sample suspected of having methylated target sequences is obtained from a biological sample, treating the sample with a reagent that can prime a portion of the target, amplifying the nucleic acid target, and comparing the degree of methylation of the amplified sample with that of a known normal sample. In yet another aspect, a sequence that is not likely to be methylated is amplified and detected for comparison with the amplified methylated sequence.

In yet another aspect, the invention provides a method for detecting a cell proliferative disorder. This is done by amplifying a gene whose methylation status is indicative of the cell proliferative disorder. The method of detecting may include contacting a nucleic acid-containing specimen or biological fluid with an agent that modifies unmethylated cytosine, amplifying the nucleic acid in the specimen with oligonucleotide primers or priming sequences that distinguish between modified methylated and nonmethylated nucleic acid, and detecting the methylated nucleic acid based on the presence or absence of amplification products produced during amplification.

The methods of the invention encompass determining a methylation ratio of a sample. The methylation ratio is a ratio between the level of methylation of a Marker (or a region of a Marker) that is hypermethylated in a diseased state relative to the level of methylation of a Marker that is not hypermethylated under the same condition (or relative to a region of the same Marker that is not hypermethylated under the same condition). When the methylation ratio is determined via comparison of methylation of a Marker that is hypermethylated in a diseased state to another Marker that is not, the second Marker can be referred to as a reference Marker.

In another aspect of the disclosure, methylation ratios are determined via quantitative real time PCR.

In another aspect of the disclosure, reporter molecules for diagnostic and/or prognostic assays are provided.

In another embodiment, the disclosure provides a kit useful for conducting an assay according to claim 1. The kit includes one or more containers; a first container containing a reagent that modifies unmethylated cytosine and a second container containing a reagent that primes amplification of CpG-containing nucleic acid, wherein the reagent distinguishes between modified methylated and nonmethylated nucleic acid.

In yet another aspect, the nucleic acid sequence that is detected in the methods of the invention or the kits for practicing them includes a promoter or a portion of a promoter.

In yet another aspect of the invention, methods and kits used to detect methylated portions of certain genes according to the claims also include steps and/or components for assaying the presence of another nucleic acid. The degree to which the purportedly methylated gene is effected by the treatment (e.g., amplification) is compared to that of the other nucleic acid to determine the extent of methylation of the Marker.

### BRIEF DESCRIPTION OF THE DRAWINGS

Fig. 1 is a graph of the results of MSPCR assays using probes and primers of the prior art.
Fig. 2 is a graph of the results of MSPCR assays using hydrolysis probes and primers according to the invention.

### DETAILED DESCRIPTION OF THE INVENTION

Hypermethylation of certain genes correlates with prostatic carcinogenesis. Nucleic acid sequences comprising such genes or portions of such genes are referred to as Markers in this specification. Markers include nucleic acids comprising the following genes or portions of such genes: GSTP1 (Seq. ID. No. 54), RARB2 (Seq. ID. No. 57), RASSF1A (Seq. ID. No. 64), TIMP3 (Seq. ID. No. 58), APC (Promoter= Seq. ID. No. 59, Gene= Seq. ID. No. 60), beta-Actin (Seq. ID. No:55 and 56) , PTGS2 (Promoter Seq. ID. No.61, Gene= Seq. ID. No. 62) and 14-3-3 sigma (Seq. ID. No.63).

Assays for detecting such hypermethylation include such techniques as MSP and restriction endonuclease analysis. The promoter region is a particularly noteworthy target for detecting such hypermethylation analysis. Sequence analysis of the promoter region of GSTP I shows that nearly 72% of the nucleotides are CG and about 10% are CpG dinucleotides.

The disclosure provides a method for determining the methylation status of certain regions of the Markers in a tissue or other biological sample of a subject in which the DNA associated with the proliferative disorder is amplified and detected. Since a decreased level of the protein encoded by the Marker (i.e., less transcription) is often the result of hypermethylation of a particular region such as the promoter, it is desirable to determine whether such regions are hypermethylated. This is seen most demonstrably in the case of the GSTP1 gene. Hypermethylated regions are those that are methylated to a statistically significant greater degree in samples from diseased tissue as compared to normal tissue.

For purposes of the invention, a nucleic acid probe or reporter specific for certain Marker regions is used to detect the presence of methylated regions of the Marker gene in biological fluids or tissues. Oligonucleotide primers based on certain portions of the Marker sequence are particularly useful for amplifying DNA by techniques such as PCR. Any specimen containing a detectable amount of the relevant polynucleotide can be used. A preferred specimen of this invention is tissue of urogenital origin, specifically, prostate tissue. Preferably the sample contains epithelial cells.

Some of the primers/probes or reporter reagents of the disclosure are used to detect methylation of expression control sequences of the Marker genes. These are nucleic acid sequences that regulate the transcription and, in some cases, translation of the nucleic acid sequence. Thus, expression control sequences can include sequences involved with promoters, enhancers, transcription terminators, start codons (i.e., ATG), splicing signals for introns, maintenance of the correct reading frame of that gene to permit proper translation of the mRNA, and stop codons.

The GSTP1 promoter is an expression control sequence exemplary of a useful Marker. It is a polynucleotide sequence that can direct transcription of the gene to produce a glutathione-s-transferase protein. The promoter region is located upstream, or 5' to the structural gene. It may include elements which are sufficient to render promoter-dependent gene expression controllable for cell-type specific, tissue-specific, or inducible by external signals or agents; such elements may be located in the 5' or 3' regions of the of the polynucleotide sequence.

One method of the disclosure includes contacting a target cell containing a Marker with a reagent that binds to the nucleic acid. The target cell component is a nucleic acid such as DNA or RNA. The reagents can include probes and primers such as PCR or MSP primers or other molecules configured to amplify and detect the target sequence. For example, the reagents can include priming sequences combined with or bonded to their own reporter segments such as those referred to as Scorpion reagents or Scorpion reporters and described in US Patents 6,326,145 and 6,270,967 to Whitcombe ***et.*** al. (incorporated herein by reference in their entirety). Though they are not the same, the terms "primers" and "priming sequences" may be used in this specification to refer to molecules or portions of molecules that prime the amplification of nucleic acid sequences.

One sensitive method of detecting methylation patterns involves combining the use of methylation-sensitive enzymes and the polymerase chain reaction (PCR). After digestion of DNA with the enzyme, PCR will amplify from primers flanking the restriction site only if DNA cleavage was prevented by methylation. Exemplary target regions to which PCR primers of the invention are designed include primers which flank the region that lies approximately between -71 and+59 bp according to genomic positioning number of M24485 (Genbank) from the transcription start site of GSTP1.

The method of the disclosure can also include contacting a nucleic acid-containing specimen with an agent that modifies unmethylated cytosine; amplifying the CpG-containing nucleic acid in the specimen by means of CpG-specific oligonucleotide primers; and detecting the methylated nucleic acid. The preferred modification is the conversion of unmethylated cytosines to another nucleotide that will distinguish the unmethylated from the methylated cytosine. Preferably, the agent modifies unmethylated cytosine to uracil and is sodium bisulfite, however, other agents that modify unmethylated cytosine, but not methylated cytosine can also be used. Sodium bisulfite (NaHSO₃) modification is most preferred and reacts readily with the 5,6-double bond of cytosine, but poorly with methylated cytosine. Cytosine reacts with the bisulfite ion to form a sulfonated cytosine reaction intermediate susceptible to deamination, giving rise to a sulfonated uracil. The sulfonate group can be removed under alkaline conditions, resulting in the formation of uracil. Uracil is recognized as a thymine by Taq polymerase and therefore upon PCR, the resultant product contains cytosine only at the position where 5-methylcytosine occurs in the starting template. Scorpion reporters and reagents and other detection systems similarly distinguish modified from unmodified species treated in this manner.

The primers used in the invention for amplification of a CpG-containing nucleic acid in the specimen, after modification (e.g., with bisulfite), specifically distinguish between untreated DNA, methylated, and non-methylated DNA. In methylation specific PCR (MSPCR), primers or priming sequences for the non-methylated DNA preferably have a T in the 3' CG pair to distinguish it from the C retained in methylated DNA, and the compliment is designed for the antisense primer. MSP primers or priming sequences for non-methylated DNA usually contain relatively few Cs or Gs in the sequence since the Cs will be absent in the sense primer and the Gs absent in the antisense primer (C becomes modified to U (uracil) which is amplified as T (thymidine) in the amplification product).

The primers of the invention are oligonucleotides of sufficient length and appropriate sequence so as to provide specific initiation of polymerization on a significant number of nucleic acids in the polymorphic locus. When exposed to the probes or reporters of the invention, the sequences that are amplified by the primers of the invention reveal methylation status and thus diagnostic information. They are more sensitive and specific than those of the prior art.

The inventive primers are most preferably eight or more deoxyribonucleotides or ribonucleotides capable of initiating synthesis of a primer extension product, which is substantially complementary to a polymorphic locus strand. Environmental conditions conducive to synthesis include the presence of nucleoside triphosphates and an agent for polymerization, such as DNA polymerase, and a suitable temperature and pH. The priming segment of the primer or priming sequence is preferably single stranded for maximum efficiency in amplification, but may be double stranded. If double stranded, the primer is first treated to separate its strands before being used to prepare extension products. The primer must be sufficiently long to prime the synthesis of extension products in the presence of the inducing agent for polymerization. The exact length of primer will depend on factors such as temperature, buffer, and nucleotide composition. The oligonucleotide primers most preferably contain about 12-20 nucleotides although they may contain more or fewer nucleotides, preferably according to well known design guidelines or rules.

Primers of the invention are designed to be substantially complementary to each strand of the genomic locus to be amplified and include the appropriate G or C nucleotides as discussed above. This means that the primers must be sufficiently complementary to hybridize with their respective strands under conditions that allow the agent for polymerization to perform. In other words, the primers should have sufficient complementarity with the 5' and 3' flanking sequence(s) to hybridize and permit amplification of the genomic locus.

The primers of the invention are employed in the amplification process. That is, reactions (preferably, an enzymatic chain reaction) that produce greater quantities of target locus relative to the number of reaction steps involved. In a most preferred embodiment, the reaction produces exponentially greater quantities of the target locus. Reactions such as these include the PCR reaction. Typically, one primer is complementary to the negative (-) strand of the locus and the other is complementary to the positive (+) strand. Annealing the primers to denatured nucleic acid followed by extension with an enzyme, such as the large fragment of DNA Polymerase I (Klenow) and nucleotides, results in newly synthesized + and - strands containing the target locus sequence. The product of the chain reaction is a discrete nucleic acid duplex with termini corresponding to the ends of the specific primers employed.

The primers of the invention may be prepared using any suitable method, such as conventional phosphotriester and phosphodiester methods including automated methods. In one such automated embodiment, diethylphosphoramidites are used as starting materials and may be synthesized as described by Beaucage, et at. (Tetrahedron Letters, 22:1859-1862, 1981). A method for synthesizing oligonucleotides on a modified solid support is described in U.S. Pat. No. 4,458,066.

Any nucleic acid specimen, in purified or non-purified form, can be utilized as the starting nucleic acid or acids, provided it contains, or is suspected of containing, the specific nucleic acid sequence containing the target locus (e.g., CpG). Thus, the process may employ, for example, DNA or RNA, including messenger RNA. The DNA or RNA may be single stranded or double stranded. In the event that RNA is to be used as a template, enzymes, and/or conditions optimal for reverse transcribing the template to DNA would be utilized. In addition, a DNA-RNA hybrid containing one strand of each may be utilized. A mixture of nucleic acids may also be employed, or the nucleic acids produced in a previous amplification reaction herein, using the same or different primers may be so utilized. The specific nucleic acid sequence to be amplified, i.e., the target locus, may be a fraction of a larger molecule or can be present initially as a discrete molecule so that the specific sequence constitutes the entire nucleic acid.

The nucleic acid-containing specimen used for detection of methylated CpG may be from any source such as tissue (particularly, prostate tissue and lymphatic tissue), blood, lymph, urine, and ejaculate and may be extracted by a variety of techniques such as that described by Maniatis, et al. (Molecular Cloning: A Laboratory Manual, Cold Spring Harbor, N.Y., pp 280, 281, 1982).

If the extracted sample is impure, it may be treated before amplification with an amount of a reagent effective to open the cells, fluids, tissues, or animal cell membranes of the sample, and to expose and/or separate the strand(s) of the nucleic acid(s). This lysing and nucleic acid denaturing step to expose and separate the strands will allow amplification to occur much more readily.

Where the target nucleic acid sequence of the sample contains two strands, it is necessary to separate the strands of the nucleic acid before it can be used as the template. Strand separation can be effected either as a separate step or simultaneously with the synthesis of the primer extension products. This strand separation can be accomplished using various suitable denaturing conditions, including physical, chemical or enzymatic means. One physical method of separating nucleic acid strands involves heating the nucleic acid until it is denatured. Typical heat denaturation may involve temperatures ranging from about 80 to 105°C for up to 10 minutes. Strand separation may also be induced by an enzyme from the class of enzymes known as helicases or by the enzyme RecA, which has helicase activity, and in the presence of riboATP, is known to denature DNA. Reaction conditions that are suitable for strand separation of nucleic acids using helicases are described by Kuhn Hoffmann-Berling (CSH-Quantitative Biology, 43:63, 1978). Techniques for using RecA are reviewed in C. Radding (Ann. Rev. Genetics, 16:405-437, 1982). Refinements of these techniques are now also well known.

When complementary strands of nucleic acid or acids are separated, regardless of whether the nucleic acid was originally double or single stranded, the separated strands are ready to be used as a template for the synthesis of additional nucleic acid strands. This synthesis is performed under conditions allowing hybridization of primers to templates to occur. Generally synthesis occurs in a buffered aqueous solution, preferably at a pH of 7-9, most preferably about 8. A molar excess (for genomic nucleic acid, usually about 10⁸:1, primer:template) of the two oligonucleotide primers is preferably added to the buffer containing the separated template strands. The amount of complementary strand may not be known if the process of the invention is used for diagnostic applications, so the amount of primer relative to the amount of complementary strand cannot always be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

The deoxyribonucleoside triphosphates dATP, dCTP, dGTP, and dTTP are added to the synthesis mixture, either separately or together with the primers, in adequate amounts and the resulting solution is heated to about 90-100°C for up to 10 minutes, preferably from 1 to 4 minutes. After this heating period, the solution is allowed to cool to room temperature, which is preferable for the primer hybridization. To the cooled mixture is added an appropriate agent for effecting the primer extension reaction (the "agent for polymerization"), and the reaction is allowed to occur under conditions known in the art. The agent for polymerization may also be added together with the other reagents if it is heat stable. This synthesis (or amplification) reaction may occur at room temperature up to a temperature at which the agent for polymerization no longer functions.

The agent for polymerization may be any compound or system that will function to accomplish the synthesis of primer extension products, preferably enzymes. Suitable enzymes for this purpose include, for example, E. coli DNA polymerase 1, Klenow fragment of E. coli DNA polymerase 1, T4 DNA polymerase, other available DNA polymerases, polymerase muteins, reverse transcriptase, and other enzymes, including heat-stable enzymes (e.g., those enzymes which perform primer extension after being subjected to temperatures sufficiently elevated to cause denaturating). A preferred agent is Taq polymerase. Suitable enzymes will facilitate combination of the nucleotides in the proper manner to form the primer extension products complementary to each locus nucleic acid strand. Generally, the synthesis will be initiated at the 3' end of each primer and proceed in the 5' direction along the template strand, until synthesis terminates, producing molecules of different lengths. There may be agents for polymerization, however, which initiate synthesis at the 5' end and proceed in the other direction, using the same process as described above.

Most preferably, the method of amplifying is by PCR. Alternative methods of amplification can also be employed as long as the methylated and non-methylated loci amplified by PCR using the primers of the invention is similarly amplified by the alternative means.

The amplified products are preferably identified as methylated or non-methylated with a probe or reporter specific to the product as described in US Patent 4,683,195 to Mullis et. al., incorporated herein by reference in its entirety. Advances in the field of probes and reporters for detecting polynucleotides are well known to those skilled in the art. Optionally, the methylation pattern of the nucleic acid can be confirmed by other techniques such as restriction enzyme digestion and Southern blot analysis. Examples of methylation sensitive restriction endonucleases which can be used to detect 5'CpG methylation include Smal, SaclI, EagI, MspI, HpaII, BstUI and BssHII.

The most preferred method of the invention involves establishing a methylation ratio according to the claims. This can be done by establishing a ratio between the amount of amplified methylated species of Marker attained and the amount of amplified reference Marker or non-methylated Marker region amplified. This is best done using quantitive realtime PCR. Ratios above an established or predetermined cutoff or threshold are considered hypermethylated and indicative of having a proliferative disorder such as cancer (prostate cancer in the case of GSTP1). Cutoffs are established according to known methods in which such methods are used for at least two sets of samples: those with known diseased conditions and those with known normal conditions. The reference Markers of the invention can also be used as internal controls. The reference Marker is preferably a gene that is constitutively expressed in the cells of the samples such as B Actin.

The inventive methods and kits can include steps and reagents for multiplexing. That is, more than one Marker can be assayed at a time.

Since a decreased level of transcription of the gene associated with the Marker is often the result of hypermethylation of the polynucleotide sequence and/or particular elements of the expression control sequences (e.g., the promoter sequence), primers prepared to match those sequences were prepared. Accordingly, the invention provides methods of detecting or diagnosing a cell proliferative disorder by detecting methylation of particular areas within the expression control or promoter region of the Markers as specified in the claims Probes useful for detecting methylation of these areas are useful in such diagnostic or prognostic methods. Preferred molecules for the detection of Markers are shown below. The short name for the Marker gene is shown in parentheses along with the type of detection system employed. Antisense only refers to the orientation of the primer that is so designated in relationship to the priming sequence of the other member of the pair with which it is associated. It is not necessarily antisense with respect to genomic DNA.
**SEQ ID NO. I (GSTP1 SCORPION):**
   CCCCGAACGTCGACCGCTCGGGG-BHQ-HEG-CGATTTCGGGGATTTTAGGGCGT
**SEQ ID NO. 2 (GSTP1 SCORPION Antisense Primer):**
   AAAATCCCGCGAACTCCCGCC
**SEQ ID NO. 3 (GSTP1 SCORPION):**
   CCCGAACGTCGACCGCTTTCGGG-BHQ-HEG-CGATTTCGGGGATTTTAGGGCGT
**SEQ ID NO. 4 (GSTP1 SCORPION Antisense Primer):**
   AAAATCCCGCGAACTCCCGCC
**SEQ ID NO. 5(GSTP1 SCORPION):**
   CCGCGGGAGTTCGCGGGCGCCG BHQ-HEG-ACTAAATCACGACGCCGACCGC
**SEQ 10 NO. 6 (GSTP1 SCORPION Antisense Primer):**
   CGGTTAGTTGCGCGGCGATTTC
**SEQ ID NO. 7 (GSTP1 SCORPION):**
   CGGGAGTTCGCGGGTCCCG-BHQ-HEG-ACTAAATCACGACGCCGACCGC
**SEQ 10 NO. 8 (GSTP1 SCORPION Antisense Primer):**
   CGGTTAGTTGCGCGGCGATTTC
**SEQ ID NO. 9(GSTP1 SCORPION):**
   GTGGTTGATGTTTGGGGTATCAACCAC-BHQ-HEG_AATCCCACAAACTCCCACCAACC
**SEQ ID NO. 10 (GSTPI SCORPION Antisense Primer):**
   GTGGTGATTTTGGGGATTTTAGGGTGT
**SEQ ID NO. 11 (GSTP1 SCORPION):**
   ACCCCAGTGGTTGATGTTTGGGGT-BHQ-HEG-AATCCCACAAACTCCCACCAACC
**SEQ ID NO. 12 (GSTPI SCORPION Antisense Primer):**
   GTGGTGATTTTGGGGATTTTAGGGTGT
**SEQ ID NO. 13 (GSTP1 SCORPION):**
**SEQ ID NO. 14 (GSTP1 SCORPION Antisense Primer):**
   TGGTTAGTTGTGTGGTGATTTTGGGGA
**SEQ ID NO. 15 (GSTP1 SCORPION):**
   CCCCGAACGTCGACCGCTCGGGG-BHQ-HEG- CGATTTCGGGGATTTTAGGGCGT
**SEQ ID NO. 16 (GSTP1 SCORPION Antisense Primer):**
   AAAATCCCGCGAACTCCCGCC
**SEQ ID NO.17 (GSTP1 SCORPION):**
**SEQ ID NO. 18 (GSTP1 SCORPION Antisense Primer):**
   AAAATCCCGCGAACTCCCGCC
**SEQ ID NO.19 (GSTP1 SCORPION):**
   CGCACGGCGAACTCCCGCCGACGTGCG BHQ-HEG-TGTAGCGGTCGTCGGGGTTG
**SEQ ID NO. 20 (GSTP1 SCORPION Antisense Primer):**
   GCCCCAATACTAAATCACGACG
**SEQ ID NO.21 (GSTP1 SCORPION):**
**SEQ ID NO. 22 (GSTP1 SCORPION Antisense Primer):**
   CACAACACCAACCACTCTTC
**SEQ ID NO.23(GSTP1 TAQMAN PRIMER):**
   CGTGATTTAGTATTGGGGCGGAGCGGGGC
**SEQ ID NO.24 (GSTP1 TAQMAN PRIMER):**
   ATCCCCGAAAAACGAACCGCGCGTA
**SEQ ID NO.25 (GSTP1 TAQMAN PROBE):**
   TCGGAGGTCGCGAGGTTTTCGTTGGA
**SEQ ID NO. 26 (RARB2 SCORPION):**
   GCCGCCGTCGAGAACGCGAGCGGGCGGC-BHQ-HEG-TACCCCGACGATACCCAAAC
**SEQ ID NO.27 (RARB2 SCORPION Antisense Primer):**
   GGGATTAGAATTTTTTATGCGAGTTGT
**SEQ ID NO.28 (RARB2 SCORPION):**
   CGGCAGGATTGGGATGTCGAGCTGCCG-B>-IQ-HEG-TACCCCGACGATACCCAAAC
**SEQ ID NO. 29 (RARB2 SCORPION Antisense Primer):**
   GGCATTAGAATTTTTTATGCGAGTTGT
**SEQ ID NO.30 (RASSF1A SCORPION):**
   GCCGCGGTTTCGTTCGGTTCGCGGC-BHQ-HEG-CCCGTACTTCGCTAACTTTAAACG
**SEQ ID NO.31 (RASSF1A SCORPION Antisense Primer):**
   GCGTTGAAGTCGGGGTTC
**SEQ ID NO. 32 (TIMP3 SCORPION):**
   GCGGCGAGTTCGGGTTGTAGCGCCGC-BHQ-HEG-CGCCTCTCCAAAATTACCGTAC
**SEQ ID NO. 33 (TIMP3 SCORPION Antisense Primer):**
   GCGTCGGAGGTTAAGGTTGTT
**SEQ ID NO. 34 (APC SCORPION):**
   GCCGGCGGGTTTTCGACGGGCCGGC-BHQ-HEG-CGAACCAAAACGCTCCCCA
**SEQ ID NO. 35 (APC SCORPION Antisense Primer):**
   GTCGGTTACGTGCGTTTATATTTAG
**SEQ ID NO. 36 (ACTIN SCORPION):**
   GCGCCCAACCGCACAAGGGCGC-BHQ-HEG-GGGTATATTTTCGAGGGGTACG
**SEQ ID NO. 37 (ACTIN SCORPION Antisense Primer):**
   CGACCCGCACTCCGCAAT
**SEQ ID NO. 38(ACTIN SCORPION):**
   CCGCGCATCACCACCCCACACGCGCGG-BHQ-HEG-GGAGTATATAGGTTGGGGAAGTTTG
**SEQ ID NO. 39 (ACTIN SCORPION Antisense Primer):**
   AACACACAATAACAAACACAAATTCAC
**SEQ ID NO. 40 (ACTIN SCORPION):**
   CCCGGCTAAACCCACCATCCAGCCGGG-BHQ-HEG-GGGAGGGTAGTTTAGTTGTGGTT
**SEQ ID NO. 41 (ACTIN SCORPION Antisense Primer):**
   CAAAACAAAAAAACTAAATCTACACAACC
**SEQ 10 NO. 42 (ACTIN SCORPION):**
   CCGCGGAACA lTCAACTCAACCGCGG-BHQ-HEG-GGAGGAGGAAGGTAGGTTTTT
**SEQ 10 NO. 43 (ACTIN SCORPION Antisense Primer):**
   ACATACAACAATCAATAACATAAAACCAC
**SEQ 10 NO. 44 (PTGS2/COX2 SCORPION):**
   CACGCCGCCGTATCTAGGCGTG-BHQ-HEG-GTTTGTTTCGACGTGATTTTTCGA
**SEQ 10 NO. 45 (PTGS2/COX2 Antisense Primer):**
   GCAAAAAATCCCCTCTCCCGC
**SEQ ID NO. 46 (PTGS2/COX2 SCORPION):**
   GCCGCGCACAAATTTCCGCGGC-BHQ-HEG-GAATTGGTTTTCGGAAGCGTTCG
**SEQ ID NO. 47 (PTGS2/COX2 Antisense Primer):**
   CCCGAATTCCACCGCC
**SEQ ID NO. 48 (PTGS2/COX2 SCORPION):**
   GGCGGAACGCACAAATTTCCGCC-BHQ-HEG-GAATTCCTTTTCGCAAGCGTTCG
**SEQ ID NO. 49 (PTGS2/COX2 Antisense Primer):**
   CCCGAATTCCACCGCC
**SEQ ID NO. 50 (PTGS2/COX2 SCORPION):**
   TGCCGCCGCCGTATCTAATGGCGGCA-BHQ-HEG-GTTTGTTTCGACGTGATTTTTTCGA
**SEQ ID NO. 51 (PTGS2/COX2 Antisense Primer):**
   GCAAAAAATCCCCTCTCCCGC
**SEQ ID NO. 52 (14-3-3 SCORPION):**
   CGGCCTTCGCTCTTCGCAAAAGGCCG-BHQ-HEG-GGTAGTTTTTATGAAAGGCGTCGTG
**SEQ ID NO. 53 (14-3-3 Antisense Primer):**
   CTAACCGCCCACCACGTT
BHQ=Black Hole Quencher (BioSearch Technologies, San Fransisco, CA)
HEG= Hexaethylene glycol

The kits of the disclosure can be configured with a variety of components provided that they all contain at least one primer or probe of the invention or a detection molecule (e.g., Scorpion reporter). In one embodiment, the kit includes reagents for amplifying and detecting hypermethylated Marker segments. Optionally, the kit includes sample preparation reagents and /or articles (e.g., tubes) to extract nucleic acids from samples.

In a preferred kit, reagents necessary for one-tube MSP are included such as, a corresponding PCR primer set, a thermostable DNA polymerase, such as Taq polymerase, and a suitable detection reagent(s) such as hydrolysis probe or molecular beacon. In optionally preferred kits, detection reagents are Scorpion reporters or reagents. A single dye primer or a fluorescent dye specific to double-stranded DNA such as ethidium bromide can also be used. The primers are preferably in quantities that yield high concentrations. Additional materials in the kit may include: suitable reaction tubes or vials, a barrier composition, typically a wax bead, optionally including magnesium; necessary buffers and reagents such as dNTPs; control nucleic acid (s) and/or any additional buffers, compounds, co-factors, ionic constituents, proteins and enzymes, polymers, and the like that may be used in MSP reactions. Optionally, the kits include nucleic acid extraction reagents and materials.

### EXAMPLES

### Example 1: Comparative

A hydrolysis probe assay was designed based upon the primers and probes in the literature and directed to the quantitative assay for detecting prostate cancer in patients with clinically localized disease. This assay includes a core CpG promoter region used to discriminate between neoplastic and non-neoplastic prostate tissue.

The primer and dual-labeled hydrolysis probe sequences tested for this design were as follows:
Forward primer GSTP1;(-192)MU17 AGTTGCGCGGCGATTTC (Seq. ID. No. 65)
Reverse primer GSTP1;(-74)ML22 GCCCCAATACTAAATCACGACG (Seq. ID. No. 20)
Probe (5'FAM/3'TAMRA)GSTP1;(-152)MP23 CGGTCGACGTTCGGGGTGTAGCG (Seq. ID. No. 66)

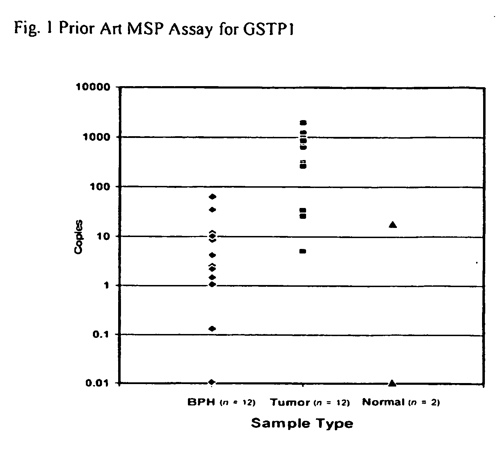

GSTP1 methylation levels were analyzed in 12 samples from benign prostatic hyperplasia (BPH), 12 samples from clinically localized prostate adenocarcinoma (Tumor), and 2 normal samples. Data is presented in total copies of GSTP1 detected and is shown in Fig. 1. The assay was found to display 75% sensitivity for detecting prostate adenocarcinoma when a cutoff was set to the highest copy number exhibited by a benign sample. The moderate distribution of GSTP1 methylation levels in prostate tissues displaying benign prostatic hyperplasia and clinically localized prostate adenocarcinoma suggests an assay that would not be clinically relevant without being complemented by another marker.

### Example 2 (Hydrolysis Probe Assay According to the Invention)

MSP primers and probe were designed to further improve sensitivity and specificity for the GSTP1 assay and were tested on the same sample set as in Example 1. This design was located further downstream of the CpG island and encompassed part of the core promoter and exon 1. The primer and dual-labeled hydrolysis probe sequences tested for this example are as follows:
Forward primer: (-71)MU29 CGTGATTTAGTATTGGGGCGGAGCGGGGC (Seq. ID No. 23)
Reverse primer: (+59)ML25 ATCCCCGAAAAACGAACCGCGCGTA (Seq. ID No. 24)
Probe (5'FAM/3'TAMRA)GSTP1;(-23)MP26 TCGGAGGTCGCGAGGTTTTCGTTGGA (Seq. ID No. 25)

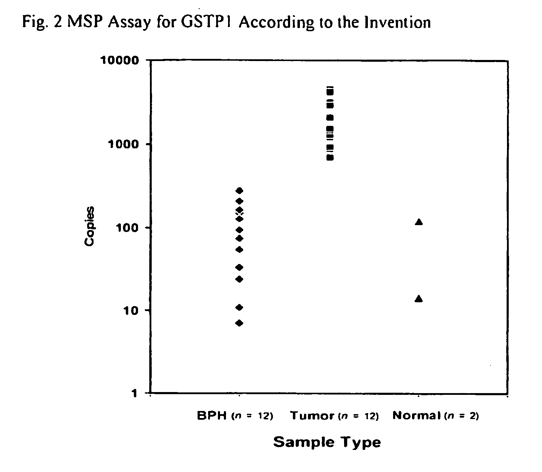

Data is presented in total copies of GSTP1 detected and is shown in Fig. 2. The assay was found to display 100% sensitivity and 100% specificity for detecting prostate adenocarcinoma when a cutoff is set to the highest copy number exhibited by a benign sample.

The 1.5kb CpG island of GSTP 1 spans through the promoter into exon 3 and has been shown to be extensively methylated throughout. Two transcription binding sites AP I and Sp1 are located just upstream of exon 1 and were the focus of the design of the probes and primers used in this example due to the high content of CpG's within SP1. This proved to be a good region for assay design due to competition between transcription factors and methyl-CpG binding proteins binding to the AP1 and Sp1 I sites in order to induce chromatin condensation and gene silencing.

### Example 3: Scorpion MSP

Scorpion reagents were designed for the following Markers and reference Markers:
Seq. ID No. 19, 20 (GSTP1)
Seq. ID No. 38, 39 (Actin)
Seq. ID No. 28, 29 (RARB2)
Seq. ID No. 30, 31 (RASSF I A)
Seq. ID No. 32, 33 (TIMP3)
Seq. ID No. 34, 35 (APC)
Seq. ID No. 50, 51 (PTGS2)
Seq. ID No. 52, 53 (14-3-3 sigma)

The designs were tested for secondary structures and inappropriate dimerizations using Oligo6 primer design software (Molecular Biology Insights, Cascade, CO, USA) and Visual OMP (DNA Software, Inc., 120½ West Washington Street Ann Arbor, MI, USA). Scorpion folding and amplicon probing was modeled using the DNA mfold program (http://bioinfo.math.rpi.edu/~mfold/dna/forml.cgi) and Visual OMP (DNA Software, Inc., 120½ West Washington Street Ann Arbor, MI, USA). Scorpions and primers were synthesized in an automated DNA synthesizer using standard phosphoramidite chemistry (Scandinavian Gene Synthesis AB, Koping, Sweden) and Biosearch Technologies (Novato, CA, USA).

### Example 3 Scorpion Reagent Testing:

The reagents described in Example 2 were tested on various human DNA samples. These samples included commercially available methylated (all cytosines preceding guanine are methylated at position 5) and unmethylated human genomic DNA (Chemicon, Temecula, CA, USA) as positive and negative template respectively. Genomic DNA from human breast cancer cell line, MCF-7 and colorectal cancer cell line, HCTI 16 as positive and negative controls for GSTP1 methylation were also used (American Type Culture Collection, Manassas, VA, USA).

Genomic DNA was modified using a commercially available sodium bisulfite conversion reagent kit (Zymo Research, Orange, CA, USA). This treatment converted all Cytosines in unmethylated DNA into Uracil, whereas in methylated DNA only cytosines not preceding guanine were converted into Uracil. All cytosines preceeding guanine (in a CpG dinucletide) remained as cytosine.

Sodium bisulfite modified genomic DNA (100 - 150 ng) was amplified in a 25 µl reaction containing the following components: 67mM Tris pH 8.8,16.6mM (NH₄)₂SO₄, 6.7mM MgCl₂, 10mM beta mercaptoethanol; 1.25mM each dATP, dCTP, dGTP, dTTP, 1 U Hot start Taq DNA Ploymerase, 250 nM Scorpion probe, 250 nM reverse or forward primer (depending on scorpion design), 625 nM of passive reference dye, ROX (for ABI7900 runs only).

The samples were then tested in a quantitative real-time PCR assay on an ABI 7900HT sequence detection system and /or Cepheid SmartCycler^{®} PCR instrument.

The PCR conditions used were:
For ABI 7900 runs:
   95°C for 5 mins.; then 40 cycles of 95°C for 30sec, 59°C for 30 sec and 72°C for 30 sec; and a final extension at 72°C for 5 mins. Optical data was collected at 59°C for every cycle.
For Cepheid smart cycler runs:
   95°C for 60 sec; then 40 cycles of 95°C for 30sec, 59°C for 30 sec, and a final extension at 72°C for 5 min. Optical data was collected at 59°C for every cycle.

The Scorpion reagents showed a PCR efficiency of 91.2% in the case of GSTP1 reagents and 95.2% in the case of Actin reagents. The average Ct values were:

| | |
|---|---|
| GSTP1 | 28 |
| B Actin | 30 |
| APC | 29 |
| TIMP3 | 30 |
| RASSF1A | 29 |
| RARB2 | 29 |
| PTGS2 | 33 |
| 14-3-3S | 32 |

### Example 4: Comparison with Molecular Beacons.

Molecular beacons were obtained for detecting GSTP1 hypermethylation along the portion of the gene described in Example 1. The beacons were applied to PCR reactions involving samples as described in Example 2. In each case, B Actin was co-amplified so that methylation ratios could be obtained. Both GSTP1 and B-actin Scorpion probe-primer sets displayed better performance in detection of methylated GSTP1 DNA and unmethylated B Actin DNA. The Ct threshold values for the designed Scorpion sets were more than 1.5 Ct better than for the molecular beacon probes (average Ct of 28.82 for beacons v/s 27.30 for Scorpion reporters). Results are as follows:

| | | | | | | |
|---|---|---|---|---|---|---|
| Universal Methylated DNA, ng | **500** | **250** | **100** | **50** | **10** | **5** |
| Scorpion (GSTP1/Bactin copy# ratio) | 272 | 310 | 285 | 296 | 339 | 288 |
| Mol Beacon (GSTP1/Baclin copy# ratio) | 759 | 735 | 758 | 767 | 797 | 814 |

### Example 5 Methylation Ratio Cutoffs

Prostate tissue samples from patients with known clinical conditions were then subjected to PCR as described in Example 2. Methylation ratios were calculated and are shown below. Data was analyzed by using the following procedure. Prior to employing an algorithm, the following pre-requisites were followed.
For Actin: CT values < 40 for both replicates (as defined by real-time PCR equipment)
For "methylated" GSTP1 result: CT values < 40 for both replicates (as defined by real-time PCR equipment)
The slope of both standard curves above -4 (PCR efficiency > 80%)
R2: 0.99, 4 relevant data points on the standard curve
Positive assay control has to generate a positive result
Routinely included no-template controls (standard curves) have to be negative If the pre-requisites were met, copy numbers were calculated for each of the genes (GSTP 1 and Actin) based on the standard curves and a ratio was calculated for each sample (including the positive control) by using the following formula: (GSTP1 Copy Number/β-Actin Copy Number ) * 1000. A cutoff was then established by using a ratio which generated high specificity and high sensitivity. The cutoff was 10 for GSTP1 to provide a sensitivity of 87% and a specificity of 100%for 142 formalin fixed paraffin embedded prostate tissues. Ratios generated were used to determine methylation status of a given sample by employing the following criteria.

| **Results** | **copy# (mGSTP1)** | **copy# (B-Actin)** | **Ratio x 1000** |
|---|---|---|---|
| **Methylated GSTP1** | >2 | >2 | >10 |
| **UnMethylated GSTP1** | any | >200 | <10 |
| **Undetermined** | <2 | <200 | |

One skilled in the art will recognize that the cutoff can be adjusted, preferably to set the sensitivity and specificity values to those suitable for the purpose for which they will be used (e.g., screening, therapy monitoring, reflex testing of ambiguous diagnoses).

### Example 6 Combination Markers (Prophetic)

Scorpion reagents for the following combinations are prepared as described in Example 1: GSTP1 Marker and RARβ2, GSTP1 and APC, and GSTP1 and PTGS2. The Markers are run in combination as multiplexed PCR according to Example 2 using repetitive runs of 50 prostate tissue samples with known conditions (i.e., normal, benign hyperplasia, prostate cancer). Expected results are shown below:

| Marker/s | Seq. ID No. | Sensitivity | Specificity |
|---|---|---|---|
| GSTP1 + RARβ2 | | 90% | 100% |
| GSTP1 + APC | | 96.2% | 92.9 |
| GSTP1 + PTGS2 | | 96.2 | 100 |

## Claims

1. A method of diagnosing the presence or predicting the course of a proliferative disorder comprising determining the methylation status of a Marker for GSTP1 in a biological sample using a nucleic acid probe and oligonucleotide primers, wherein the oligonucleotide probe is encoded by SEQ ID NO:25 and the oligonucleotide primers are encoded by SEQ ID NOs: 23 and 24.

2. The method according to claim 1 further comprising measuring the presence of a reference Marker.

3. The method according to claim 2 wherein the reference Marker is selected from the group consisting of B Actin.

4. The method of claim 1 wherein one or more further Markers are used.

5. The method of claim 4 wherein the one or more further Markers includes a Marker for APC, TIMP, RASSF1A, RARB2, PTGS2, or 14-3-3.

6. The method according to claim 4 wherein reagents for the one or more further Markers include a member of the group consisting of Seq. ID. No. 26-35 and 44-53.

7. The method of claim 1 wherein the sample is prostate tissue.

8. The method of claim 1 wherein the sample is urine, serum, plasma, or circulating cells.

9. A composition comprising all of the members of the group consisting of Seq. ID No. 23-25.

10. A kit for conducting an assay according to claim 1, comprising: nucleic acid amplification and detection reagents comprising all the members of the group consisting of Seq. ID No. 23-25.

11. The kit of claim 10 wherein the amplification and detection reagents are Seq. ID No. 23-25 together with reagents selected from the group consisting of Seq. ID No. 36-43.

12. The kit of claim 10 wherein the reagents detect the hypermethylation of GSTP 1.

13. The kit of claim 10 further comprising reagents for amplifying and detecting the presence of a constitutively expressed gene.

14. The kit of claim 10 further comprising reagents for amplifying and detecting the presence of constitutively expressed genes.

15. The method of claim 1 further comprising establishing a methylation ratio and determining whether the methylation ratio exceeds a cutoff value.

16. The method of claim 1 for use in therapy monitoring.

17. The method of claim 1 for use in screening.

18. The method of claim 1 for use in resolving ambiguous or indeterminate results of cancer assays obtained using methods different from claim 1.

## Patentansprüche

1. Verfahren zur Diagnose des Vorliegens oder zur Vorhersage des Verlaufs einer proliferativen Erkrankung, wobei man den Methylierungszustand eines Markers für GSTP1 in einer biologischen Probe unter Verwendung einer Nukleinsäuresonde und von Oligonukleotid-Primern bestimmt, wobei die Oligonukleotidsonde durch SEQ ID NO: 25 und die Oligonukleotid-Primer durch SEQ ID NO: 23 und 24 codiert ist bzw. sind.

2. Verfahren gemäß Anspruch 1, bei dem man ferner das Vorliegen eines Referenzmarkers misst.

3. Verfahren gemäß Anspruch 2, wobei der Referenzmarker aus der aus B-Actin bestehenden Gruppe ausgewählt ist.

4. Verfahren nach Anspruch 1, wobei ein weiterer oder mehrere weitere Marker verwendet wird bzw. werden.

5. Verfahren nach Anspruch 4, wobei der eine weitere bzw. die mehreren weiteren Marker einen Marker für APC, TIMP, RASSF1A, RARB2, PTGS2 oder 14-3-3 umfasst bzw. umfassen.

6. Verfahren gemäß Anspruch 4, wobei Reagentien für den einen weiteren bzw. die mehreren weiteren Marker ein Mitglied der aus Seq. ID Nr. 26-35 und 44-53 bestehenden Gruppe umfassen.

7. Verfahren nach Anspruch 1, wobei es sich bei der Probe um Prostatagewebe handelt.

8. Verfahren nach Anspruch 1, wobei es sich bei der Probe um Urin, Serum, Plasma oder zirkulierende Zellen handelt.

9. Zusammensetzung, umfassend alle Mitglieder der aus Seq. ID Nr. 23-25 bestehenden Gruppe.

10. Kit zur Durchführung eines Testverfahrens gemäß Anspruch 1, umfassend: Nukleinsäureamplifikations-und -nachweisreagentien, umfassend alle Mitglieder der aus Seq. ID Nr. 23-25 bestehenden Gruppe.

11. Kit nach Anspruch 10, wobei es sich bei den Amplifikations- und Nachweisreagentien um Seq. ID Nr. 23-25 zusammen mit aus der aus Seq. ID Nr. 36-43 bestehenden Gruppe ausgewählten Reagentien handelt.

12. Kit nach Anspruch 10, wobei die Reagentien die Hypermethylierung von GSTP1 nachweisen.

13. Kit nach Anspruch 10, ferner umfassend Reagentien zur Amplifikation und zum Nachweis des Vorliegens eines konstitutiv exprimierten Gens.

14. Kit nach Anspruch 10, ferner umfassend Reagentien zur Amplifikation und zum Nachweis des Vorliegens konstitutiv exprimierter Gene.

15. Verfahren nach Anspruch 1, bei dem man ferner ein Methylierungsverhältnis ermittelt und bestimmt, ob das Methylierungsverhältnis einen Ausschlussgrenzwert überschreitet.

16. Verfahren nach Anspruch 1 zur Verwendung bei der Therapieüberwachung.

17. Verfahren nach Anspruch 1 zur Verwendung beim Screening.

18. Verfahren nach Anspruch 1 zur Verwendung bei der Auflösung mit von Anspruch 1 verschiedenen Verfahren erhaltener zweideutiger oder unbestimmter Krebstestergebnisse.

## Revendications

1. Procédé pour diagnostiquer la présence ou prédire l'évolution d'un trouble prolifératif, comprenant la détermination de l'état de méthylation d'un marqueur pour le GSTP1 dans un échantillon biologique par utilisation d'une sonde à acide nucléique et d'amorces oligonucléotidiques, la sonde oligonucléotidique étant codée par SEQ ID N° 25, et les amorces oligonucléotidiques étant codées par SEQ ID N° 23 et 24.

2. Procédé selon la revendication 1, qui comprend en outre la mesure de la présence d'un marqueur de référence.

3. Procédé selon la revendication 2, dans lequel le marqueur de référence est choisi dans le groupe consistant en la B-actine.

4. Procédé de la revendication 1, dans lequel on utilise un ou plusieurs marqueurs supplémentaires.

5. Procédé de la revendication 4, dans lequel le ou les marqueurs supplémentaires comprennent un marqueur pour l'APC, le TIMP, le RASSF1A, le RARB2, le PTGS2 ou le 14-3-3.

6. Procédé selon la revendication 4, dans lequel les réactifs pour le ou les marqueurs supplémentaires comprennent un membre du groupe consistant en SEQ ID N° 26-35 et 44-53.

7. Procédé de la revendication 1, dans lequel l'échantillon est un tissu prostatique.

8. Procédé de la revendication 1, dans lequel l'échantillon est l'urine, le sérum, le plasma ou les cellules circulantes.

9. Composition comprenant tous les membres du groupe consistant en SEQ ID N° 23-25.

10. Trousse pour mettre en oeuvre un essai selon la revendication 1, comprenant des réactifs d'amplification et de détection d'acides nucléiques, comprenant tous les membres du groupe consistant en SEQ ID N° 23-25.

11. Trousse de la revendication 10, dans laquelle les réactifs d'amplification et de détection sont SEQ ID N° 23-25, en même temps que les réactifs choisis dans le groupe consistant en SEQ ID N° 36-43.

12. Trousse de la revendication 10, dans laquelle les réactifs détectent l'hyperméthylation du GSTP1.

13. Trousse de la revendication 10, qui comprend en outre des réactifs pour amplifier et détecter la présence d'un gène à expression constitutive.

14. Trousse de la revendication 10, qui comprend en outre des réactifs pour amplifier et détecter la présence de gènes à expression constitutive.

15. Procédé de la revendication 1, qui comprend en outre l'établissement d'un taux de méthylation, et le fait de déterminer si le taux de méthylation dépasse une valeur de coupure.

16. Procédé de la revendication 1, pour utilisation en suivi de thérapie.

17. Procédé de la revendication 1, pour utilisation en criblage.

18. Procédé de la revendication 1, pour utilisation afin de statuer sur des résultats ambigus ou indéterminés provenant de tests du cancer, obtenus par utilisation de procédés différents de celui de la revendication 1.


### SEQUENCE LISTING

<110> VENER, Tatiana
   MEHROTRA, Jyoti
   VARDE, Shobha
   MAZUMDER, Abhijit
   BADEN, Jon
   BACKUS, John
<120> DETECTING GENE METHYLATION
<130> VDX5027USNP
<140> 60/717790
   <141> > 2005-09-15
<160> 66
<170> PatentIn version 3.3
<210> 1
   <211> 46
   <212> DNA
   <213> Artificial
<220>
   <223> GSTP1 Scorpion
<220>
   <221 > modified_base
   <222> (23)..(23)
   <223> -BHQ-HEG-
<220>
   <221> modified_base
   <222> (23)..(23)
   <223> -BHQ (Black Hole Quencher) -HEG (Hexaethylglycol) -
<400> 1
   ccccgaacgt cgaccgctcg gggcgatttc ggggatttta gggcgt 46
<210> 2
   <211> 21
   <212> DNA
   <213> Artificial
<220>
   <223> GSTP1 Scorpion Antisense Primer
<400> 2
   aaaatcccgc gaactcccgc c 21
<210> 3
   <211> 46
   <212> DNA
   <213> Artificial
<220>
   <223> GSTP1 Scorpion
<220>
   <221> modified_base
   <222> (23)..(23)
   <223> -BHQ-HEG-
<400> 3
   cccgaacgtc gaccgctttc gggcgatttc ggggatttta gggcgt 46
<210> 4
   <211> 21
   <212> DNA
   <213> Artificial
<220>
   <223> GSTP1 Scorpion Antisense Primer
<400> 4
   aaaatcccgc gaactcccgc c 21
<210> 5
   <211> 44
   <212> DNA
   <213> Artificial
<220>
   <223> GSTP1 Scorpion
<220>
   <221 > modified_base
   <222> (22)..(22)
   <223> -BHQ-HEG-
<400> 5
   cggcgggagt tcgcgggcgc cgactaaatc acgacgccga ccgc 44
<210> 6
   <211> 22
   <212> DNA
   <213> Artificial
<220>
   <223> GSTP1 Scorpion Antisense Primer
<400> 6
   cggttagttg cgcggcgatt tc 22
<210> 7
   <211> 41
   <212> DNA
   <213> Artificial
<220>
   <223> GSTP1 Scorpion
<220>
   <221> modified_base
   <222> (19)..(19)
   <223> -BHQ-HEG-
<220>
   <221> modified_base
   <222> (25)..(25)
   <223> -BHQ-HEG-
<400> 7
   cgggagttcg cgggtcccga ctaaatcacg acgccgaccg c 41
<210> 8
   <211> 22
   <212> DNA
   <213> Artificial
<220>
   <223> GSTP1 Scorpion Antisense Primer
<400> 8
   cggttagttg cgcggcgatt tc 22
<210> 9
   <211> 50
   <212> DNA
   <213> Artificial
<220>
   <223> GSTP1 Scorpion
<220>
   <221> modified_base
   <222> (27)..(27)
   <223> -BHQ-HEG-
<400> 9
   gtggttgatg tttggggtat caaccacaat cccacaaact cccaccaacc 50
<210> 10
   <211> 27
   <212> DNA
   <213> Artificial
<220>
   <223> GSTP1 Scorpion Antisense Primer
<400> 10
   gtggtgattt tggggatttt agggtgt 27
<210> 11
   <211> 47
   <212> DNA
   <213> Artificial
<220>
   <223> GSTP1 Scorpion
<220>
   <221> modified_base
   <222> (24)..(24)
   <223> -BHQ-HEG-
<400> 11
   accccagtgg ttgatgtttg gggtaatccc acaaactccc accaacc 47
<210> 12
   <211> 27
   <212> DNA
   <213> Artificial
<220>
   <223> GSTP1 Scorpion
<400> 12
   gtggtgattt tggggatttt agggtgt 27
<210> 13
   <211> 56
   <212> DNA
   <213> Artificial
<220>
   <223> GSTP1 Scorpion
<220>
   <221> modified_base
   <222> (28)..(28)
   <223> -BHQ-HEG-
<400> 13
   ccccacaggt tggtgggagt ttgtggggcc caatactaaa tcacaacacc aaccac 56
<210> 14
   <211> 27
   <212> DNA
   <213> Artificial
<220>
   <223> GSTP1 Scorpion Antisense Primer
<400> 14
   tggttagttg tgtggtgatt ttgggga 27
<210> 15
   <211> 46
   <212> DNA
   <213> Artificial
<220>
   <223> GSTP1 Scorpion
<220>
   <221> modified_base
   <222> (23)..(23)
   <223> -BHQ-HEG-
<400> 15
   ccccgaacgt cgaccgctcg gggcgatttc ggggatttta gggcgt 46
<210> 16
   <211> 21
   <212> DNA
   <213> Artificial
<220>
   <223> GSTP1 Scorpion Antisense Primer
<400> 16
   aaaatcccgc gaactcccgc c 21
<210> 17
   <211> 53
   <212> DNA
   <213> Artificial
<220>
   <223> GSTP1 Scorpion
<220>
   <221 modified_base
   <222> (30)..(30)
   <223> -BHQ-HEG-
<400> 17
   cgcacgccga acgtcgaccg caaacgtgcg cgatttcggg gattttaggg cgt 53
<210> 18
   <211> 21
   <212> DNA
   <213> Artificial
<220>
   <223> GSTP1 Scorpion Antisense Primer
<400> 18
   aaaatcccgc gaactcccgc c 21
<210> 19
   <211> 47
   <212> DNA
   <213> Artificial
<220>
   <223> GSTP1 Scorpion
<220>
   <221> modified_base
   <222> (27)..(27)
   <223> -BHQ-HEG-
<400> 19
   cgcacggcga actcccgccg acgtgcgtgt agcggtcgtc ggggttg 47
<210> 20
   <211> 22
   <212> DNA
   <213> Artificial
<220>
   <223> GSTP1 Scorpion Antisense Primer
<400> 20
   gccccaatac taaatcacga cg 22
<210> 21
   <211> 55
   <212> DNA
   <213> Artificial
<220>
   <223> GSTP1 Scorpion
<220>
   <221> modified_base
   <222> (34)..(34)
   <223> -BHQ-HEG-
<400> 21
   ccgacgcaca aaaaaacacc ctaaaatccg tcggggttag ttgtgtggtg atttt 55
<210> 22
   <211> 20
   <212> DNA
   <213> Artificial
<220>
   <223> GSTP1 Scorpion Antisense Primer
<400> 22
   cacaacacca accactcttc 20
<210> 23
   <211> 29
   <212> DNA
   <213> Artificial
<220>
   <223> GSTP1 Taqman Primer
<400> 23
   cgtgatttag tattggggcg gagcggggc 29
<210> 24
   <211> 25
   <212> DNA
   <213> Artificial
<220>
   <223> GSTP1 Taqman Primer
<400> 24
   atccccgaaa aacgaaccgc gcgta 25
<210> 25
   <211> 26
   <212> DNA
   <213> Artificial
<220>
   <223> GSTP1 Taqman Probe
<400> 25
   tcggaggtcg cgaggttttc gttgga 26
<210> 26
   <211> 48
   <212> DNA
   <213> Artificial
<220>
   <223> RARB2 Scorpion
<220>
   <221> modified_base
   <222> (28)..(28)
   <223> -BHQ-HEG-
<400> 26
   gccgccgtcg agaacgcgag cgggcggcta ccccgacgat acccaaac 48
<210> 27
   <211> 27
   <212> DNA
   <213> Artificial
<220>
   <223> RARB2 Scorpion Antisense Primer
<400> 27
   gggattagaa ttttttatgc gagttgt 27
<210> 28
   <211> 47
   <212> DNA
   <213> Artificial
<220>
   <223> RARB2 Scorpion
<220>
   <221> modified_base
   <222> (27)..(27)
   <223> -BHQ-HEG-
<400> 28
   cggcaggatt gggatgtcga gctgccgtac cccgacgata cccaaac 47
<210> 29
   <211> 27
   <212> DNA
   <213> Artificial
<220>
   <223> RARB2 Scorpion Antisense Primer
<400> 29
   gggattagaa ttttttatgc gagttgt 27
<210> 30
   <211> 49
   <212> DNA
   <213> Artificial
<220>
   <223> RASSFIA Scorpion
<220>
   <221> modified_base
   <222> (25)..(25)
   <223> -BHQ-HEG-
<400> 30
   gccgcggttt cgttcggttc gcggccccgt acttcgctaa ctttaaacg 49
<210> 31
   <211> 18
   <212> DNA
   <213> Artificial
<220>
   <223> RASSFIA Scorpion Antisense Primer
<400> 31
   gcgttgaagt cggggttc 18
<210> 32
   <211> 48
   <212> DNA
   <213> Artificial
<220>
   <223> TIMP3 Scorpion
<220>
   <221> modified_base
   <222> (26)..(26)
   <223> -BHQ-HEG-
<400> 32
   gcggcgagtt cgggttgtag cgccgccgcc tctccaaaat taccgtac 48
<210> 33
   <211> 21
   <212> DNA
   <213> Artificial
<220>
   <223> TIMP3 Scorpion Antisense Primer
<400> 33
   gcgtcggagg ttaaggttgt t 21
<210> 34
   <211> 44
   <212> DNA
   <213> Artificial
<220>
   <223> APC Scorpion
<220>
   <221> modified_base
   <222> (25)..(25)
   <223> -BHQ-HEG-
<400> 34
   gccggcgggt tttcgacggg ccggccgaac caaaacgctc ccca 44
<210> 35
   <211> 25
   <212> DNA
   <213> Artificial
<220>
   <223> APC Scorpion Antisense Primer
<400> 35
   gtcggttacg tgcgtttata tttag 25
<210> 36
   <211> 44
   <212> DNA
   <213> Artificial
<220>
   <223> Actin Scorpion
<220>
   <221> modified_base
   <222> (22)..(22)
   <223> -BHQ-HEG-
<400> 36
   gcgcccaacc gcacaagggc gcgggtatat tttcgagggg tacg 44
<210> 37
   <211> 18
   <212> DNA
   <213> Artificial
<220>
   <223> ACTIN Scorpion Antisense Primer
<400> 37
   cgacccgcac tccgcaat 18
<210> 38
   <211> 52
   <212> DNA
   <213> Artificial
<220>
   <223> ACTIN Scorpion
<220>
   <221> modified_base
   <222> (27)..(27)
   <223> -BHQ-HEG-
<400> 38
   ccgcgcatca ccaccccaca cgcgcgggga gtatataggt tggggaagtt tg 52
<210> 39
   <211> 27
   <212> DNA
   <213> Artificial
<220>
   <223> ACTIN Scorpion Antisense Primer
<400> 39
   aacacacaat aacaaacaca aattcac 27
<210> 40
   <211> 50
   <212> DNA
   <213> Artificial
<220>
   <223> ACTIN Scorpion
<220>
   <221> modified_base
   <222> (27)..(27)
   <223> -BHQ-HEG-
<400> 40
   cccggctaaa cccaccatcc agccgggggg agggtagttt agttgtggtt 50
<210> 41
   <211> 29
   <212> DNA
   <213> Artificial
<220>
   <223> ACTIN Scorpion Antisense Primer
<400> 41
   caaaacaaaa aaactaaatc tacacaacc 29
<210> 42
   <211> 47
   <212> DNA
   <213> Artificial
<220>
   <223> ACTIN Scorpion
<220>
   <221> modified_base
   <222> (26)..(26)
   <223> -BHQ-HEG-
<400> 42
   ccgcggaaca ttcaactcaa ccgcggggag gaggaaggta ggttttt 47
<210> 43
   <211> 29
   <212> DNA
   <213> Artificial
<220>
   <223> ACTIN Scorpion Antisense Primer
<400> 43
   acatacaaca atcaataaca taaaaccac 29
<210> 44
   <211> 47
   <212> DNA
   <213> Artificial
<220>
   <223> PTGS2/COX2 Scorpion
<220>
   <221> modified_base
   <222> (22)..(22)
   <223> -BHQ-HEG-
<400> 44
   cacgccgccg tatctaggcg tggtttgttt cgacgtgatt ttttcga 47
<210> 45
   <211> 21
   <212> DNA
   <213> Artificial
<220>
   <223> PTGS2/COX2 Antisense Primer
<400> 45
   gcaaaaaatc ccctctcccg c 21
<210> 46
   <211> 45
   <212> DNA
   <213> Artificial
<220>
   <223> PTGS2/COX2 Scorpion
<220>
   <221> modified_base
   <222> (22)..(22)
   <223> -BHQ-HEG-
<400> 46
   gccgcgcaca aatttccgcg gcgaattggt tttcggaagc gttcg 45
<210> 47
   <211> 16
   <212> DNA
   <213> Artificial
<220>
   <223> PTGS2/COX2 Antisense Primer
<400> 47
   cccgaattcc accgcc 16
<210> 48
   <211> 46
   <212> DNA
   <213> Artificial
<220>
   <223> PTGS2/COX2 Scorpion
<220>
   <221> modified_base
   <222> (23)..(23)
   <223> -BHQ-HEG-
<400> 48
   ggcggaacgc acaaatttcc gccgaattgg ttttcggaag cgttcg 46
<210> 49
   <211> 16
   <212> DNA
   <213> Artificial
<220>
   <223> PTGS2/COX2 Antisense Primer
<400> 49
   cccgaattcc accgcc 16
<210> 50
   <211> 51
   <212> DNA
   <213> Artificial
<220>
   <223> PTGS2/COX2 Scorpion
<220>
   <221> modified_base
   <222> (26)..(26)
   <223> -BHQ-HEG-
<400> 50
   tgccgccgcc gtatctaatg gcggcagttt gtttcgacgt gattttttcg a 51
<210> 51
   <211> 21
   <212> DNA
   <213> Artificial
<220>
   <223> PTGS2/COX2 Antisense Primer
<400> 51
   gcaaaaaatc ccctctcccg c 21
<210> 52
   <211> 51
   <212> DNA
   <213> Artificial
<220>
   <223> 14-3-3 Scorpion
<220>
   <221> modified_base
   <222> (26)..(26)
   <223> -BHQ-HEG-
<400> 52
   cggccttcgc tcttcgcaaa aggccgggta gtttttatga aaggcgtcgt 51
<210> 53
   <211> 18
   <212> DNA
   <213> Artificial
<220>
   <223> 14-3-3 Antisense Primer
<400> 53
   ctaaccgccc accacgtt 18
<210> 54
   <211> 4260
   <212> DNA
   <213> Homo sapiens
<220>
   <221> misc_feature
   <223> >gi|341173|gb|M24485.1|HUMGSTP1G Homo sapiens (clone pHGST-pi) glutathione S-transferase pi (GSTP1) gene, complete cds
<400> 54
   aacaagagat caatatctag aataaatgga gatctgcaaa tcaacagaaa gtaggcagca 60
   aagccaaaga aaatagccta aggcacagcc actaaaagga acgtgatcat gtcctttgca 120
   gggacatggg tggagctgga agccgttagc ctcagcaaac tcacacagga acagaaaacc 180
   agcgagaccg catggtctca cttataagtg ggagctgaac aatgagaaca catggtcaca 240
   tggcggcgat caacacacac tggtgcctgt tgagcggggt gctggggagg gagagtacca 300
   ggaagaatag ctaagggata ctgggcttaa tacctgggtg atgggatgat ctgtacagca 360
   aaccatcatg gcgcacacac ctatgtaaca aacctgcaca tcctgcacat gtaccccaga 420
   acttcaaata aaagttggac ggccaggcgt ggtggctcac gcctgtaatc ccagcacttt 480
   gggaagccga ggcgtgcaga tcacctaagg tcaggagttc gagaccagcc cggccaacat 540
   ggtgaaaccc cgtctctact aaaaatacaa aaatcagcca gatgtggcac gcacctataa 600
   ttccacctac tcgggaggct gaagcagaat tgcttgaacc cgagaggcgg aggttgcagt 660
   gagccgccga gatcgcgcca ctgcactcca gcctgggcca cagcgtgaga ctacgtcata 720
   aaataaaata aaataacaca aaataaaata aaataaaata aaataaaata aaataataaa 780
   ataaaataaa ataaaataaa ataaaataaa ataaagcaat ttcctttcct ctaagcggcc 840
   tccacccctc tcccctgccc tgtgaagcgg gtgtgcaagc tccgggatcg cagcggtctt 900
   agggaatttc cccccgcgat gtcccggcgc gccagttcgc tgcgcacact tcgctgcggt 960
   cctcttcctg ctgtctgttt actccctagg ccccgctggg gacctgggaa agagggaaag 1020
   gcttccccgg ccagctgcgc ggcgactccg gggactccag ggcgcccctc tgcggccgac 1080
   gcccggggtg cagcggccgc cggggctggg gccggcggga gtccgcggga ccctccagaa 1140
   gagcggccgg cgccgtgact cagcactggg gcggagcggg gcgggaccac ccttataagg 1200
   ctcggaggcc gcgaggcctt cgctggagtt tcgccgccgc agtcttcgcc accagtgagt 1260
   acgcgcggcc cgctccccgg ggatggggct cagagctccc agcatggggc caacccgcag 1320
   catcaggccc gggctcccgg cagggctcct cgcccacctc gagacccggg acgggggcct 1380
   aggggaccca ggacgtcccc agtgccgtta gcggctttca gggggcccgg agcgcctcgg 1440
   ggagggatgg gaccccgggg gcggggaggg ggggcaggct gcgctcaccg cgccttggca 1500
   tcctcccccg ggctccagca aacttttctt tgttcgctgc agtgccgccc tacaccgtgg 1560
   tctatttccc agttcgaggt aggagcatgt gtctggcagg gaagggaggc aggggctggg 1620
   gctgcagccc acagcccctc gcccacccgg agagatccga acccccttat ccctccgtcg 1680
   tgtggctttt accccgggcc tccttcctgt tccccgcctc tcccgccatg cctgctcccc 1740
   gccccagtgt tgtgtgaaat cttcggagga acctgtttac ctgttccctc cctgcactcc 1800
   tgacccctcc ccgggttgct gcgaggcgga gtcggcccgg tccccacatc tcgtacttct 1860
   ccctccccgc aggccgctgc gcggccctgc gcatgctgct ggcagatcag ggccagagct 1920
   ggaaggagga ggtggtgacc gtggagacgt ggcaggaggg ctcactcaaa gcctcctgcg 1980
   taagtgacca tgcccgggca aggggagggg gtgctgggcc ttagggggct gtgactagga 2040
   tcgggggacg cccaagctca gtgcccctcc ctgagccatg cctcccccaa cagctatacg 2100
   ggcagctccc caagttccag gacggagacc tcaccctgta ccagtccaat accatcctgc 2160
   gtcacctggg ccgcaccctt ggtgagtctt gaacctccaa gtccagggca ggcatgggca 2220
   agcctctgcc cccggagccc ttttgtttaa atcagctgcc ccgcagccct ctggagtgga 2280
   ggaaactgag acccactgag gttacgtagt ttgcccaagg tcaagcctgg gtgcctgcaa 2340
   tccttgccct gtgccaggct gcctcccagg tgtcaggtga gctctgagca cctgctgtgt 2400
   ggcagtctct catccttcca cgcacatcct cttcccctcc tcccaggctg gggctcacag 2460
   acagccccct ggttggccca tccccagtga ctgtgtgttg atcaggcgcc cagtcacgcg 2520
   gcctgctccc ctccacccaa ccccagggct ctatgggaag gaccagcagg aggcagccct 2580
   ggtggacatg gtgaatgacg gcgtggagga cctccgctgc aaatacatct ccctcatcta 2640
   caccaactat gtgagcatct gcaccagggt tgggcactgg gggctgaaca aagaaagggg 2700
   cttcttgtgc cctcaccccc cttacccctc aggtggcttg ggctgacccc ttcttgggtc 2760
   agggtgcagg ggctgggtca gctctgggcc aggggcccag gggcctggga caagacacaa 2820
   cctgcaccct tattgcctgg gacatcaacc agccaagtaa cgggtcatgg gggcgagtgc 2880
   aaggacagag acctccagca actggtggtt tctgatctcc tggggtggcg agggcttcct 2940
   ggagtagcca gaggtggagg aggatttgtc gccagtttct ggatggaggt gctggcactt 3000
   ttagctgagg aaaatatgca gacacagagc acatttgggg acctgggacc agttcagcag 3060
   aggcagcgtg tgtgcgcgtg cgtgtgcgtg tgtgtgcgtg tgtgtgtgta cgcttgcatt 3120
   tgtgtcgggt gggtaaggag atagagatgg gcgggcagta ggcccaggtc ccgaaggcct 3180
   tgaacccact ggtttggagt ctcctaaggg caatgggggc cattgagaag tctgaacagg 3240
   gctgtgtctg aatgtgaggt ctagaaggat cctccagaga agccagctct aaagcttttg 3300
   caatcatctg gtgagagaac ccagcaagga tggacaggca gaatggaata gagatgagtt 3360
   ggcagctgaa gtggacagga tttggtacta gcctggttgt ggggagcaag cagaggagaa 3420
   tctgggactc tggtgtctgg cctggggcag acgggggtgt ctcaggggct gggagggatg 3480
   agagtaggat gatacatggt ggtgtctggc aggaggcggg caaggatgac tatgtgaagg 3540
   cactgcccgg gcaactgaag ccttttgaga ccctgctgtc ccagaaccag ggaggcaaga 3600
   ccttcattgt gggagaccag gtgagcatct ggccccatgc tgttccttcc tcgccaccct 3660
   ctgcttccag atggacacag gtgtgagcca tttgtttagc aaagcagagc agacctaggg 3720
   gatgggctta ggccctctgc ccccaattcc tccagcctgc tcccgctggc tgagtcccta 3780
   gcccccctgc cctgcagatc tccttcgctg actacaacct gctggacttg ctgctgatcc 3840
   atgaggtcct agcccctggc tgcctggatg cgttccccct gctctcagca tatgtggggc 3900
   gcctcagtgc ccggcccaag ctcaaggcct tcctggcctc ccctgagtac gtgaacctcc 3960
   ccatcaatgg caacgggaaa cagtgagggt tggggggact ctgagcggga ggcagagttt 4020
   gccttccttt ctccaggacc aataaaattt ctaagagagc tactatgagc actgtgtttc 4080
   ctgggacggg gcttaggggt tctcagcctc gaggtcggtg ggagggcaga gcagaggact 4140
   agaaaacagc tcctccagca cagtcagtgg cttcctggag ccctcagcct ggctgtgttt 4200
   actgaacctc acaaactaga agaggaagaa aaaaaaagag agagagaaac aaagagaaat 4260
<210> 55
   <211> 2011
   <212> DNA
   <213> Homo sapiens
<220>
   <221> misc_feature
   <223> B-Actin
   >gi|28337|emb|Y00474.1|HSACTBPR Human beta-actin gene 5'-flanking region, CpG Island 1656 to 1955
<400> 55
   gagctctgtc tcttggccag ctgaatggag gcccagcggc aacacaggtc ctgcctgggg 60
   atcaggtctg ctctgcaccc caccttgctg cctggagccg cccacctgac aacctctcat 120
   ccctgctctg tagatccggt cccatcccca ctgcccaccc caccccccca gcactccacc 180
   cagttcaacg ttccacgaac ccccagaacc agccctcatc aacaggcagc aagaagggcc 240
   ccccgcccat cgccccacaa cgccagccgg gtgaactgta gcgttggcag gtcctgaggc 300
   agctgaaaga tacaaggcca gggacaggac agtcccatcc ccaggaggca gggagtatac 360
   aggctgggga agtttgccct tgcgtggggt ggtgatggag gaggctcagc aagtcttctg 420
   gactgtgaac ctgtgtctgc cactgtgtgc tgggtggtgg tcatctttcc caccaggctg 480
   tggcctctgc aaccttcaag ggaggagcag gtcccattgg ctgagcacag ccttgtacgt 540
   gaactgaaca agcagcctcc ttcctggcca caggttccat gtccttatat ggactcatct 600
   ttgcctattg cgacacacac tcaatgaaca cctactacgc gctgcaaaga gccccgcagg 660
   cctgaggtgc ccccacctca ccactcttcc tatttttgtg taaaaatcca gcttcttgtc 720
   accacctcca aggaggggga ggaggaggaa ggcaggttcc tctaggctga gccgaatgcc 780
   cctctgtggt cccacgccac tgatcgctgc atgcccacca cctgggtaca cacagtctgt 840
   gattcccgga gcagaacgga ccctgcccac ccggtcttgt gtgctactca gtggacagac 900
   ccaaggcaag aaagggtgac aaggacaggg tcttcccagg ctggctttga gttcctagca 960
   ccgccccgcc cccaatcctc tgtggcacat ggagtcttgg tccccagagt cccccagcgg 1020
   cctccagatg gtctgggagg gcagttcagc tgtggctgcg catagcagac atacaacgga 1080
   cggtgggccc agacccaggc tgtgtagacc cagccccccc gccccgcagt gcctaggtca 1140
   cccactaacg ccccaggcct ggtcttggct gggcgtgact gttaccctca aaagcaggca 1200
   gctccagggt aaaaggtgcc ctgccctgta gagcccactt ccttcccagg gctgcggctg 1260
   ggtaggtttg tagccttcat cacgggccac ctccagccac tggaccgctg gcccctgccc 1320
   tgtcctgggg agtgtggtcc tgcgactcta atggccgcaa gccacctgac tcccccaaca 1380
   ccacactcta cctctcaagc ccaggtctct ccctagtgac ccacccagca catttagcta 1440
   gctgagcccc acagccagag gtcctcaggc cctgctttca gggcagttgc tctgaagtcg 1500
   gcaaggggga gtgactgcct ggccactcca tgccctccaa gagctccttc tgcaggagcg 1560
   tacagaaccc agggccctgg cacccgtgca gaccctggcc caccccacct gggcgctcag 1620
   tgcccaagag atgtccacac ctaggatgtc ccgcggtggg tggggggccc gagagacggg 1680
   caggccgggg gcaggcctgg ccatgcgggg ccgaaccggg cactgcccag cgtggggcgc 1740
   gggggccacg gcgcgcgccc ccagcccccg ggcccagcac cccaaggcgg ccaacgccaa 1800
   aactctccct cctcctcttc ctcaatctcg ctctcgctct tttttttttt cgcaaaagga 1860
   ggggagaggg ggtaaaaaaa tgctgcactg tcggcgaagc cggtgagtga gcggcgcggg 1920
   gccaatcgcg tgcgccgttc cgaaagttgc cttttatggc tcgagcggcc gcggcggcgc 1980
   cctataaaac ccagcggcgc gacgcgccac c 2011
<210> 56
   <211> 1792
   <212> DNA
   <213> Homo sapiens
<220>
   <221> misc_feature
   <223> >gi|5016088|ref|NM_001101.2| Homo sapiens actin, beta (ACTB), mRNA
<400> 56
   cgcgtccgcc ccgcgagcac agagcctcgc ctttgccgat ccgccgcccg tccacacccg 60
   ccgccagctc accatggatg atgatatcgc cgcgctcgtc gtcgacaacg gctccggcat 120
   gtgcaaggcc ggcttcgcgg gcgacgatgc cccccgggcc gtcttcccct ccatcgtggg 180
   gcgccccagg caccagggcg tgatggtggg catgggtcag aaggattcct atgtgggcga 240
   cgaggcccag agcaagagag gcatcctcac cctgaagtac cccatcgagc acggcatcgt 300
   caccaactgg gacgacatgg agaaaatctg gcaccacacc ttctacaatg agctgcgtgt 360
   ggctcccgag gagcaccccg tgctgctgac cgaggccccc ctgaacccca aggccaaccg 420
   cgagaagatg acccagatca tgtttgagac cttcaacacc ccagccatgt acgttgctat 480
   ccaggctgtg ctatccctgt acgcctctgg ccgtaccact ggcatcgtga tggactccgg 540
   tgacggggtc acccacactg tgcccatcta cgaggggtat gccctccccc atgccatcct 600
   gcgtctggac ctggctggcc gggacctgac tgactacctc atgaagatcc tcaccgagcg 660
   cggctacagc ttcaccacca cggccgagcg ggaaatcgtg cgtgacatta aggagaagct 720
   gtgctacgtc gccctggact tcgagcaaga gatggccacg gctgcttcca gctcctccct 780
   ggagaagagc tacgagctgc ctgacggcca ggtcatcacc attggcaatg agcggttccg 840
   ctgccctgag gcactcttcc agccttcctt cctgggcatg gagtcctgtg gcatccacga 900
   aactaccttc aactccatca tgaagtgtga cgtggacatc cgcaaagacc tgtacgccaa 960
   cacagtgctg tctggcggca ccaccatgta ccctggcatt gccgacagga tgcagaagga 1020
   gatcactgcc ctggcaccca gcacaatgaa gatcaagatc attgctcctc ctgagcgcaa 1080
   gtactccgtg tggatcggcg gctccatcct ggcctcgctg tccaccttcc agcagatgtg 1140
   gatcagcaag caggagtatg acgagtccgg cccctccatc gtccaccgca aatgcttcta 1200
   ggcggactat gacttagttg cgttacaccc tttcttgaca aaacctaact tgcgcagaaa 1260
   acaagatgag attggcatgg ctttatttgt tttttttgtt ttgttttggt tttttttttt 1320
   tttttggctt gactcaggat ttaaaaactg gaacggtgaa ggtgacagca gtcggttgga 1380
   gcgagcatcc cccaaagttc acaatgtggc cgaggacttt gattgcacat tgttgttttt 1440
   ttaatagtca ttccaaatat gagatgcatt gttacaggaa gtcccttgcc atcctaaaag 1500
   ccaccccact tctctctaag gagaatggcc cagtcctctc ccaagtccac acaggggagg 1560
   tgatagcatt gctttcgtgt aaattatgta atgcaaaatt tttttaatct tcgccttaat 1620
   acttttttat tttgttttat tttgaatgat gagccttcgt gccccccctt cccccttttt 1680
   gtcccccaac ttgagatgta tgaaggcttt tggtctccct gggagtgggt ggaggcagcc 1740
   agggcttacc tgtacactga cttgagacca gttgaataaa agtgcacacc tt 1792
<210> 57
   <211> 2762
   <212> DNA
   <213> Homo sapiens
<220>
   <221> misc_feature
   <223> RARB2
   >gi|14916495|ref|NM_016152.2| Homo sapiens retinoic acid receptor, beta (RARB), transcript variant 2, mRNA
<400> 57
   gtgacagaag tagtaggaag tgagctgttc agaggcagga gggtctattc tttgccaaag 60
   gggggaccag aattccccat gcgagctgtt tgaggactgg gatgccgaga acgcgagcga 120
   tccgagcagg gtttgtctgg gcaccgtcgg ggtaggatcc ggaacgcatt cggaaggctt 180
   tttgcaagca tttacttgga aggagaactt gggatctttc tgggaacccc ccgccccggc 240
   tggattggcc gagcaagcct ggaaaatgca attgaaacac agagcaccag ctctgaggaa 300
   ctcgtcccaa gccccccatc tccacttcct ccccctcgag tgtacaaacc ctgcttcgtc 360
   tgccaggaca aatcatcagg gtaccactat ggggtcagcg cctgtgaggg atgtaagggc 420
   tttttccgca gaagtattca gaagaatatg atttacactt gtcaccgaga taagaactgt 480
   gttattaata aagtcaccag gaatcgatgc caatactgtc gactccagaa gtgctttgaa 540
   gtgggaatgt ccaaagaatc tgtcaggaat gacaggaaca agaaaaagaa ggagacttcg 600
   aagcaagaat gcacagagag ctatgaaatg acagctgagt tggacgatct cacagagaag 660
   atccgaaaag ctcaccagga aactttccct tcactctgcc agctgggtaa atacaccacg 720
   aattccagtg ctgaccatcg agtccgactg gacctgggcc tctgggacaa attcagtgaa 780
   ctggccacca agtgcattat taagatcgtg gagtttgcta aacgtctgcc tggtttcact 840
   ggcttgacca tcgcagacca aattaccctg ctgaaggccg cctgcctgga catcctgatt 900
   cttagaattt gcaccaggta taccccagaa caagacacca tgactttctc agacggcctt 960
   accctaaatc gaactcagat gcacaatgct ggatttggtc ctctgactga ccttgtgttc 1020
   acctttgcca accagctcct gcctttggaa atggatgaca cagaaacagg ccttctcagt 1080
   gccatctgct taatctgtgg agaccgccag gaccttgagg aaccgacaaa agtagataag 1140
   ctacaagaac cattgctgga agcactaaaa atttatatca gaaaaagacg acccagcaag 1200
   cctcacatgt ttccaaagat cttaatgaaa atcacagatc tccgtagcat cagtgctaaa 1260
   ggtgcagagc gtgtaattac cttgaaaatg gaaattcctg gatcaatgcc acctctcatt 1320
   caagaaatgc tggagaattc tgaaggacat gaacccttga ccccaagttc aagtgggaac 1380
   acagcagagc acagtcctag catctcaccc agctcagtgg aaaacagtgg ggtcagtcag 1440
   tcaccactcg tgcaataaga cattttctag ctacttcaaa cattccccag taccttcagt 1500
   tccaggattt aaaatgcaag aaaaaacatt tttactgctg cttagttttt ggactgaaaa 1560
   gatattaaaa ctcaagaagg accaagaagt tttcatatgt atcaatatat atactcctca 1620
   ctgtgtaact tacctagaaa tacaaacttt tccaatttta aaaaatcagc catttcatgc 1680
   aaccagaaac tagttaaaag cttctatttt cctctttgaa cactcaagat gcatggcaaa 1740
   gacccagtca aaatgattta cccctggtta agtttctgaa gactttgtac atacagaagt 1800
   atggctctgt tctttctata ctgtatgttt ggtgctttcc ttttgtcttg catactcaaa 1860
   ataaccatga caccaaggtt atgaaataga ctactgtaca cgtctaccta ggttcaaaaa 1920
   gataactgtc ttgctttcat ggaatagtca agacatcaag gtaaggaaac aggactattg 1980
   acaggactat tgtacagtat gacaagataa ggctgaagat attctacttt agttagtatg 2040
   gaagcttgtc tttgctcttt ctgatgctct caaactgcat cttttatttc atgttgccca 2100
   gtaaaagtat acaaattccc tgcactagca gaagagaatt ctgtatcagt gtaactgcca 2160
   gttcagttaa tcaaatgtca tttgttcaat tgttaatgtc actttaaatt aaaagtggtt 2220
   tattacttgt ttaatgacat aactacacag ttagttaaaa aaaatttttt tacagtaatg 2280
   atagcctcca aggcagaaac acttttcagt gttaagtttt tgtttacttg ttcacaagcc 2340
   attagggaaa tttcatggga taattagcag gctggtctac cactggacca tgtaactcta 2400
   gtgtccttcc tgattcatgc ctgatattgg gatttttttc cagcccttct tgatgccaag 2460
   ggctaattat attacatccc aaagaaacag gcatagaatc tgcctccttt gaccttgttc 2520
   aatcactatg aagcagagtg aaagctgtgg tagagtggtt aacagataca agtgtcagtt 2580
   tcttagttct catttaagca ctactggaat tttttttttt gatatattag caagtctgtg 2640
   atgtactttc actggctctg tttgtacatt gagattgttt gtttaacaat gctttctatg 2700
   ttcatatact gtttaccttt ttccatggac tctcctggca aagaataaaa tatatttatt 2760
   tt 2762
<210> 58
   <211> 5487
   <212> DNA
   <213> Homo sapiens
<220>
   <221> misc_feature
   <223> TIMP3
   >gi|21536431|ref|NM_000362.3| Homo sapiens tissue inhibitor of metalloproteinase 3 (Sorsby fundus dystrophy, pseudoinflammatory) (TIMP3), mRNA
<400> 58
   tctgtcgact tgccccagag ctgatccttg tctttgtcca cttctcagcg aggatggcac 60
   ttcagggagc ccttccctta ctatcgcaga gagagcaggc cctccccagt catgtccaac 120
   ccagaactct gttttgtttt cttcatagcc ctagcatcac agaaaatcac cctgtgcatt 180
   catggatgtc cacgggggca agggctttgt gttgcttaac ccagcatcct gaaccgtgtt 240
   tgttgaatga atacagaacc ccgtttgctc tgggagagca cagaaaacag tcttctatca 300
   tatatcatag ccagctgcaa acagcagatg gcttcccata tcccagagag taagaaccag 360
   agagagagag aaagagagag agtttgggtc tttctcctct gtgcctgctc tctccagaga 420
   aactggaggg gtagcagtta gcattccccc gctggttcca ccaagcacag tcaaggtctc 480
   taggacatgg ccacccctca cctgtggaag cggtcctgct ggggtgggtg ggtgttagtt 540
   ggttctggtt tgggtcagag acacccagtg gcccaggtgg gcgtggggcc agggcgcaga 600
   cgagaagggg cacgagggct ccgctccgag gacccagcgg caagcaccgg tcccgggcgc 660
   gccccagccc acccactcgc gtgcccacgg cggcattatt ccctataagg atctgaacga 720
   tccgggggcg gccccgcccc gttacccctt gcccccggcc ccgccccctt tttggagggc 780
   cgatgaggta atgcggctct gccattggtc tgagggggcg ggccccaaca gcccgaggcg 840
   gggtccccgg gggcccagcg ctatatcact cggccgccca ggcagcggcg cagagcgggc 900
   agcaggcagg cggcgggcgc tcagacggct tctcctcctc ctcttgctcc tccagctcct 960
   gctccttcgc cgggaggccg cccgccgagt cctgcgccag cgccgaggca gcctcgctgc 1020
   gccccatccc gtcccgccgg gcactcggag ggcagcgcgc cggaggccaa ggttgccccg 1080
   cacggcccgg cgggcgagcg agctcgggct gcagcagccc cgccggcggc gcgcacggca   1140
   actttggaga ggcgagcagc agccccggca gcggcggcag cagcggcaat gaccccttgg   1200
   ctcgggctca tcgtgctcct gggcagctgg agcctggggg actggggcgc cgaggcgtgc 1260
   acatgctcgc ccagccaccc ccaggacgcc ttctgcaact ccgacatcgt gatccgggcc 1320
   aaggtggtgg ggaagaagct ggtaaaggag gggcccttcg gcacgctggt ctacaccatc 1380
   aagcagatga agatgtaccg aggcttcacc aagatgcccc atgtgcagta catccacacg 1440
   gaagcttccg agagtctctg tggccttaag ctggaggtca acaagtacca gtacctgctg 1500
   acaggtcgcg tctatgatgg caagatgtac acggggctgt gcaacttcgt ggagaggtgg 1560
   gaccagctca ccctctccca gcgcaagggg ctgaactatc ggtatcacct gggttgtaac 1620
   tgcaagatca agtcctgcta ctacctgcct tgctttgtga cttccaagaa cgagtgtctc 1680
   tggaccgaca tgctctccaa tttcggttac cctggctacc agtccaaaca ctacgcctgc 1740
   atccggcaga agggcggcta ctgcagctgg taccgaggat gggccccccc ggataaaagc 1800
   atcatcaatg ccacagaccc ctgagcgcca gaccctgccc cacctcactt ccctcccttc 1860
   ccgctgagct tcccttggac actaactctt cccagatgat gacaatgaaa ttagtgcctg 1920
   ttttcttgca aatttagcac ttggaacatt taaagaaagg tctatgctgt catatggggt 1980
   ttattgggaa ctatcctcct ggccccaccc tgccccttct ttttggtttt gacatcattc 2040
   atttccacct gggaatttct ggtgccatgc cagaaagaat gaggaacctg tattcctctt 2100
   cttcgtgata atataatctc tattttttta ggaaaacaaa aatgaaaaac tactccattt 2160
   gaggattgta attcccaccc ctcttgcttc ttccccacct caccatctcc cagaccctct 2220
   tccctttgcc cttctcctcc aatacataaa ggacacagac aaggaacttg ctgaaaggcc 2280
   aaccatttca ggatcagtca aaggcagcaa gcagatagac tcaaggtgtg tgaaagatgt 2340
   tatacaccag gagctgccac tgcatgtccc aaccagactg tgtctgtctg tgtctgcatg 2400
   taagagtgag ggagggaagg aaggaactac aagagagtcg gagatgatgc agcacacaca 2460
   caattcccca gcccagtgat gcttgtgttg accagatgtt cctgagtctg gagcaagcac 2520
   ccaggccaga ataacagagc tttcttagtt ggtgaagact taaacatctg cctgaggtca 2580
   ggaggcaatt tgcctgcctt gtacaaaagc tcaggtgaaa gactgagatg aatgtctttc 2640
   ctctccctgc ctcccaccag acttcctcct ggaaaacgct ttggtagatt tggccaggag 2700
   ctttctttta tgtaaattgg ataaatacac acaccataca ctatccacag atatagccaa 2760
   gtagatttgg gtagaggata ctatttccag aatagtgttt agctcaccta gggggatatg 2820
   tttgtataca catttgcata tacccacatg gggacataag ctaatttttt tacaggacac 2880
   agaattctgt tcaatgctgt taaatatgcc aatagtttaa tctcttctat tttgttgtcg 2940
   ttgcttgttt gaagaaaatc atgacattcc aagttgacat ttttttttca ttttaattaa 3000
   aatttgaaat tctgaacacc gtcagcaccc tctcttccct atcatgggtc atctgacccc 3060
   tgtccgtctc cttgtccctg cttcatgttt gggggccttt ctttaactgc cttcctggct 3120
   tagctcagat ggcagatgag agtgtagtca agggcctggg cacaggaggg agagctgcag 3180
   agtgtcctgc ctgccttggc tggagggaca cctctcctgg gtgtggagac agcttggttc 3240
   cctttcccta gctccctggt gggtgaatgc cacctcctga gatcctcacc tcttggaatt 3300
   aaaattgttg gtcactgggg aaagcctgag tttgcaacca gttgtagggt ttctgttgtg 3360
   tttttttttt tttttttgaa ataaaactat aatataaatt ctcctattaa ataaaattat 3420
   tttaagtttt agtgtcaaaa gtgagatgct gagagtaggt gataatgtat attttacaga 3480
   gtgggggttg gcaggatggt gacattgaac atgattgctc tctgtctctt ttttcagctt 3540
   atgggtattt atcttctatt agtatttgta tcttcagttc attccacttt aggaaacaga 3600
   gctgccaatt gaaacagaag aagaaaaaaa aaaaaagcag cagacaacac actgtagagt 3660
   cttgcacaca cacaagtgcc caggcaaggt gcttggcaga accgcagagt gggaagagag 3720
   taccggcatc gggtttcctt gggatcaatt tcattaccgt gtacctttcc cattgtggtc 3780
   atgccatttg gcagggggag aatgggaggc ttggccttct ttgtgaggca gtgtgagcag 3840
   aagctgatgc cagcatgtca ctggttttga agggatgagc ccagacttga tgttttggga 3900
   ttgtccttat tttaacctca aggtctcgca tggtggggcc cctgaccaac ctacacaagt 3960
   tccctcccac aagtggacat cagtgtcttc tctgtgaggc atctggccat tcgcactccc 4020
   tggtgtggtc agcctctctc acacaaggag gaacttgggt gaaggctgag tgtgaggcac 4080
   ctgaagtttc cctgcggagt cgataaatta gcagaaccac atccccatct gttaggcctt 4140
   ggtgaggagg ccctgggcaa agaagggtct ttcgcaaagc gatgtcagag ggcggttttg 4200
   agctttctat aagctatagc tttgtttatt tcacccgttc acttactgta taatttaaaa 4260
   tcatttatgt agctgagaca cttctgtatt tcaatcatat catgaacatt ttattttgct 4320
   aaatcttgtg tcatgtgtag gctgtaatat gtgtacattg tgtttaagag aaaaatgaaa 4380
   cccacatgcc gccattttcc tgaatcaaat tctgcagtgg aatggagagg aaaatacttc 4440
   taggcaagca gctagactgg tgaattgggg gaaatagaag gaactagtaa ctgagactcc 4500
   tccagcctcc tccctattgg aatcccaatg gctcctggag taggaaaaaa gtttaaacta 4560
   cattcatgtt cttgttctgt gtcactcggc cctgggtagt ctaccattta cttcacccca 4620
   agtcctgctg cccatccagt tgggaagcca tgattttcct aagaatccag ggccatggga 4680
   gatacaattc caagttctcg cttcctcctt tgggcatctc ttctgcctcc caatcaagga 4740
   agctccatgc tcaggctctc agctctcggg ccagtgctct gctctgtcca gggtaggtaa 4800
   tactgggaga ctcctgtctt ttaccctccc ctcgttccag acctgcctca tggtggcaac 4860
   atggttcttg aacaattaaa gaaacaaatg actttttgga atagccctgt ctagggcaaa 4920
   ctgtggcccc caggagacac tacccttcca tgccccagac ctctgtcttg catgtgacaa 4980
   ttgacaatct ggactacccc aagatggcac ccaagtgttt ggcttctggc tacctaaggt 5040
   taacatgtca ctagagtatt tttatgagag acaaacatta taaaaatctg atggcaaaag 5100
   caaaacaaaa tggaaagtag gggaggtgga tgtgacaaca acttccaaat tggctctttg 5160
   gaggcgagag gaaggggaga acttggagaa tagtttttgc tttgggggta gaggcttctt 5220
   agattctccc agcatccgcc tttcccttta gccagtctgc tgtcctgaaa cccagaagtg 5280
   atggagagaa accaacaaga gatctcgaac cctgtctaga aggaatgtat ttgttgctaa 5340
   atttcgtagc actgtttaca gttttcctcc atgttattta tgaattttat attccgtgaa 5400
   tgtatattgt cttgtaatgt tgcataatgt tcacttttta tagtgtgtcc tttattctaa 5460
   acagtaaagt ggttttattt ctatcac 5487
<210> 59
   <211> 866
   <212> DNA
   <213> Homo sapiens
<220>
   <221> misc_feature
   <223> APC
   >gi|551463|gb|U02509.1|HSU02509 Human adenomatous polyposis coli (APC) gene, promoter sequence
<220>
   <221> misc_feature
   <222> (315)..(315)
   <223> n is a, c, g, or t
<400> 59
   acttatatat ctgacagttg atttgtcctc acctctaaat tggaatttaa gcatcacctg 60
   gttcgattta atgcaatgta gaatttgcat taaaatacta cattaaagcc tcagatttgt 120
   agtagctaac agcacttcta tgtatgtgtc agggactgct ctaaatactt catatatatt 180
   aactcctcta ttctgtactt ctgttcccgt tttatacagc aggaaattga aacactgaga 240
   ggttaagtaa ctaaagttac agagctagag tgacaggagt aaagcttcaa ctcaggcaac 300
   ccagacgtcc agagntctga tctccactac taagctgcta gcatagcttt tctggtaact 360
   atttttaatt caatataatt cgaatgatct atctaacaag tcatcactct gacaactcag 420
   tgacttgtaa tgtaaaatta ttcattgtaa ttcacttaat attattgttt ctctgtgctg 480
   caaaaatcat agcaatcgag atgtaattta ttactctccc tcccacctcc ggcatcttgt 540
   gctaatcctt ctgccctgcg gacctccccc gactctttac tatgcgtgtc aactgccatc 600
   aacttccttg cttgctgggg actggggccg tgagggcata cccccgaggg gtacggggct 660
   agggctaggc aggctgtgcg gttgggcggg gccctgtgcc ccactgcgga gtgcgggtcg 720
   ggaagcggag agagaagcag ctgtgtaatc cgctggatgc ggaccagggc gctccccatt 780
   cccgtcggga gcccgccgat tggctgggtg tgggcgcacg tgaccgacat gtggctgtat 840
   tggtgcagcc cgccagggtg tcactg 866
<210> 60
   <211> 10386
   <212> DNA
   <213> Homo sapiens
<220>
   <221> misc_feature
   <223> >gi|21626462|ref|NM_000038.2| Homo sapiens adenomatosis polyposis coli (APC), mRNA
<220>
   <221> misc_feature
   <222> (9521)..(9521)
   <223> n is a, c, g, or t
<400> 60
   attgaggact cggaaatgag gtccaagggt agccaaggat ggctgcagct tcatatgatc 60
   agttgttaaa gcaagttgag gcactgaaga tggagaactc aaatcttcga caagagctag 120
   aagataattc caatcatctt acaaaactgg aaactgaggc atctaatatg aaggaagtac 180
   ttaaacaact acaaggaagt attgaagatg aagctatggc ttcttctgga cagattgatt 240
   tattagagcg tcttaaagag cttaacttag atagcagtaa tttccctgga gtaaaactgc 300
   ggtcaaaaat gtccctccgt tcttatggaa gccgggaagg atctgtatca agccgttctg 360
   gagagtgcag tcctgttcct atgggttcat ttccaagaag agggtttgta aatggaagca 420
   gagaaagtac tggatattta gaagaacttg agaaagagag gtcattgctt cttgctgatc 480
   ttgacaaaga agaaaaggaa aaagactggt attacgctca acttcagaat ctcactaaaa 540
   gaatagatag tcttccttta actgaaaatt tttccttaca aacagatatg accagaaggc 600
   aattggaata tgaagcaagg caaatcagag ttgcgatgga agaacaacta ggtacctgcc 660
   aggatatgga aaaacgagca cagcgaagaa tagccagaat tcagcaaatc gaaaaggaca 720
   tacttcgtat acgacagctt ttacagtccc aagcaacaga agcagagagg tcatctcaga 780
   acaagcatga aaccggctca catgatgctg agcggcagaa tgaaggtcaa ggagtgggag 840
   aaatcaacat ggcaacttct ggtaatggtc agggttcaac tacacgaatg gaccatgaaa 900
   cagccagtgt tttgagttct agtagcacac actctgcacc tcgaaggctg acaagtcatc 960
   tgggaaccaa ggtggaaatg gtgtattcat tgttgtcaat gcttggtact catgataagg 1020
   atgatatgtc gcgaactttg ctagctatgt ctagctccca agacagctgt atatccatgc 1080
   gacagtctgg atgtcttcct ctcctcatcc agcttttaca tggcaatgac aaagactctg 1140
   tattgttggg aaattcccgg ggcagtaaag aggctcgggc cagggccagt gcagcactcc 1200
   acaacatcat tcactcacag cctgatgaca agagaggcag gcgtgaaatc cgagtccttc 1260
   atcttttgga acagatacgc gcttactgtg aaacctgttg ggagtggcag gaagctcatg 1320
   aaccaggcat ggaccaggac aaaaatccaa tgccagctcc tgttgaacat cagatctgtc 1380
   ctgctgtgtg tgttctaatg aaactttcat ttgatgaaga gcatagacat gcaatgaatg 1440
   aactaggggg actacaggcc attgcagaat tattgcaagt ggactgtgaa atgtacgggc 1500
   ttactaatga ccactacagt attacactaa gacgatatgc tggaatggct ttgacaaact 1560
   tgacttttgg agatgtagcc aacaaggcta cgctatgctc tatgaaaggc tgcatgagag 1620
   cacttgtggc ccaactaaaa tctgaaagtg aagacttaca gcaggttatt gcaagtgttt 1680
   tgaggaattt gtcttggcga gcagatgtaa atagtaaaaa gacgttgcga gaagttggaa 1740
   gtgtgaaagc attgatggaa tgtgctttag aagttaaaaa ggaatcaacc ctcaaaagcg 1800
   tattgagtgc cttatggaat ttgtcagcac attgcactga gaataaagct gatatatgtg 1860
   ctgtagatgg tgcacttgca tttttggttg gcactcttac ttaccggagc cagacaaaca 1920
   ctttagccat tattgaaagt ggaggtggga tattacggaa tgtgtccagc ttgatagcta 1980
   caaatgagga ccacaggcaa atcctaagag agaacaactg tctacaaact ttattacaac 2040
   acttaaaatc tcatagtttg acaatagtca gtaatgcatg tggaactttg tggaatctct 2100
   cagcaagaaa tcctaaagac caggaagcat tatgggacat gggggcagtt agcatgctca 2160
   agaacctcat tcattcaaag cacaaaatga ttgctatggg aagtgctgca gctttaagga 2220
   atctcatggc aaataggcct gcgaagtaca aggatgccaa tattatgtct cctggctcaa 2280
   gcttgccatc tcttcatgtt aggaaacaaa aagccctaga agcagaatta gatgctcagc 2340
   acttatcaga aacttttgac aatatagaca atttaagtcc caaggcatct catcgtagta 2400
   agcagagaca caagcaaagt ctctatggtg attatgtttt tgacaccaat cgacatgatg 2460
   ataataggtc agacaatttt aatactggca acatgactgt cctttcacca tatttgaata 2520
   ctacagtgtt acccagctcc tcttcatcaa gaggaagctt agatagttct cgttctgaaa 2580
   aagatagaag tttggagaga gaacgcggaa ttggtctagg caactaccat ccagcaacag 2640
   aaaatccagg aacttcttca aagcgaggtt tgcagatctc caccactgca gcccagattg 2700
   ccaaagtcat ggaagaagtg tcagccattc atacctctca ggaagacaga agttctgggt 2760
   ctaccactga attacattgt gtgacagatg agagaaatgc acttagaaga agctctgctg 2820
   cccatacaca ttcaaacact tacaatttca ctaagtcgga aaattcaaat aggacatgtt 2880
   ctatgcctta tgccaaatta gaatacaaga gatcttcaaa tgatagttta aatagtgtca 2940
   gtagtagtga tggttatggt aaaagaggtc aaatgaaacc ctcgattgaa tcctattctg 3000
   aagatgatga aagtaagttt tgcagttatg gtcaataccc agccgaccta gcccataaaa 3060
   tacatagtgc aaatcatatg gatgataatg atggagaact agatacacca ataaattata 3120
   gtcttaaata ttcagatgag cagttgaact ctggaaggca aagtccttca cagaatgaaa 3180
   gatgggcaag acccaaacac ataatagaag atgaaataaa acaaagtgag caaagacaat 3240
   caaggaatca aagtacaact tatcctgttt atactgagag cactgatgat aaacacctca 3300
   agttccaacc acattttgga cagcaggaat gtgtttctcc atacaggtca cggggagcca 3360
   atggttcaga aacaaatcga gtgggttcta atcatggaat taatcaaaat gtaagccagt 3420
   ctttgtgtca agaagatgac tatgaagatg ataagcctac caattatagt gaacgttact 3480
   ctgaagaaga acagcatgaa gaagaagaga gaccaacaaa ttatagcata aaatataatg 3540
   aagagaaacg tcatgtggat cagcctattg attatagttt aaaatatgcc acagatattc 3600
   cttcatcaca gaaacagtca ttttcattct caaagagttc atctggacaa agcagtaaaa 3660
   ccgaacatat gtcttcaagc agtgagaata cgtccacacc ttcatctaat gccaagaggc 3720
   agaatcagct ccatccaagt tctgcacaga gtagaagtgg tcagcctcaa aaggctgcca 3780
   cttgcaaagt ttcttctatt aaccaagaaa caatacagac ttattgtgta gaagatactc 3840
   caatatgttt ttcaagatgt agttcattat catctttgtc atcagctgaa gatgaaatag 3900
   gatgtaatca gacgacacag gaagcagatt ctgctaatac cctgcaaata gcagaaataa 3960
   aagaaaagat tggaactagg tcagctgaag atcctgtgag cgaagttcca gcagtgtcac 4020
   agcaccctag aaccaaatcc agcagactgc agggttctag tttatcttca gaatcagcca 4080
   ggcacaaagc tgttgaattt tcttcaggag cgaaatctcc ctccaaaagt ggtgctcaga 4140
   cacccaaaag tccacctgaa cactatgttc aggagacccc actcatgttt agcagatgta 4200
   cttctgtcag ttcacttgat agttttgaga gtcgttcgat tgccagctcc gttcagagtg 4260
   aaccatgcag tggaatggta agtggcatta taagccccag tgatcttcca gatagccctg 4320
   gacaaaccat gccaccaagc agaagtaaaa cacctccacc acctcctcaa acagctcaaa 4380
   ccaagcgaga agtacctaaa aataaagcac ctactgctga aaagagagag agtggaccta 4440
   agcaagctgc agtaaatgct gcagttcaga gggtccaggt tcttccagat gctgatactt 4500
   tattacattt tgccacggaa agtactccag atggattttc ttgttcatcc agcctgagtg 4560
   ctctgagcct cgatgagcca tttatacaga aagatgtgga attaagaata atgcctccag 4620
   ttcaggaaaa tgacaatggg aatgaaacag aatcagagca gcctaaagaa tcaaatgaaa 4680
   accaagagaa agaggcagaa aaaactattg attctgaaaa ggacctatta gatgattcag 4740
   atgatgatga tattgaaata ctagaagaat gtattatttc tgccatgcca acaaagtcat 4800
   cacgtaaagc aaaaaagcca gcccagactg cttcaaaatt acctccacct gtggcaagga 4860
   aaccaagtca gctgcctgtg tacaaacttc taccatcaca aaacaggttg caaccccaaa 4920
   agcatgttag ttttacaccg ggggatgata tgccacgggt gtattgtgtt gaagggacac 4980
   ctataaactt ttccacagct acatctctaa gtgatctaac aatcgaatcc cctccaaatg 5040
   agttagctgc tggagaagga gttagaggag gagcacagtc aggtgaattt gaaaaacgag 5100
   ataccattcc tacagaaggc agaagtacag atgaggctca aggaggaaaa acctcatctg 5160
   taaccatacc tgaattggat gacaataaag cagaggaagg tgatattctt gcagaatgca 5220
   ttaattctgc tatgcccaaa gggaaaagtc acaagccttt ccgtgtgaaa aagataatgg 5280
   accaggtcca gcaagcatct gcgtcgtctt ctgcacccaa caaaaatcag ttagatggta 5340
   agaaaaagaa accaacttca ccagtaaaac ctataccaca aaatactgaa tataggacac 5400
   gtgtaagaaa aaatgcagac tcaaaaaata atttaaatgc tgagagagtt ttctcagaca 5460
   acaaagattc aaagaaacag aatttgaaaa ataattccaa ggacttcaat gataagctcc 5520
   caaataatga agatagagtc agaggaagtt ttgcttttga ttcacctcat cattacacgc 5580
   ctattgaagg aactccttac tgtttttcac gaaatgattc tttgagttct ctagattttg 5640
   atgatgatga tgttgacctt tccagggaaa aggctgaatt aagaaaggca aaagaaaata 5700
   aggaatcaga ggctaaagtt accagccaca cagaactaac ctccaaccaa caatcagcta 5760
   ataagacaca agctattgca aagcagccaa taaatcgagg tcagcctaaa cccatacttc 5820
   agaaacaatc cacttttccc cagtcatcca aagacatacc agacagaggg gcagcaactg 5880
   atgaaaagtt acagaatttt gctattgaaa atactccagt ttgcttttct cataattcct 5940
   ctctgagttc tctcagtgac attgaccaag aaaacaacaa taaagaaaat gaacctatca 6000
   aagagactga gccccctgac tcacagggag aaccaagtaa acctcaagca tcaggctatg 6060
   ctcctaaatc atttcatgtt gaagataccc cagtttgttt ctcaagaaac agttctctca 6120
   gttctcttag tattgactct gaagatgacc tgttgcagga atgtataagc tccgcaatgc 6180
   caaaaaagaa aaagccttca agactcaagg gtgataatga aaaacatagt cccagaaata 6240
   tgggtggcat attaggtgaa gatctgacac ttgatttgaa agatatacag agaccagatt 6300
   cagaacatgg tctatcccct gattcagaaa attttgattg gaaagctatt caggaaggtg 6360
   caaattccat agtaagtagt ttacatcaag ctgctgctgc tgcatgttta tctagacaag 6420
   cttcgtctga ttcagattcc atcctttccc tgaaatcagg aatctctctg ggatcaccat 6480
   ttcatcttac acctgatcaa gaagaaaaac cctttacaag taataaaggc ccacgaattc 6540
   taaaaccagg ggagaaaagt acattggaaa ctaaaaagat agaatctgaa agtaaaggaa 6600
   tcaaaggagg aaaaaaagtt tataaaagtt tgattactgg aaaagttcga tctaattcag 6660
   aaatttcagg ccaaatgaaa cagccccttc aagcaaacat gccttcaatc tctcgaggca 6720
   ggacaatgat tcatattcca ggagttcgaa atagctcctc aagtacaagt cctgtttcta 6780
   aaaaaggccc accccttaag actccagcct ccaaaagccc tagtgaaggt caaacagcca 6840
   ccacttctcc tagaggagcc aagccatctg tgaaatcaga attaagccct gttgccaggc 6900
   agacatccca aataggtggg tcaagtaaag caccttctag atcaggatct agagattcga 6960
   ccccttcaag acctgcccag caaccattaa gtagacctat acagtctcct ggccgaaact 7020
   caatttcccc tggtagaaat ggaataagtc ctcctaacaa attatctcaa cttccaagga 7080
   catcatcccc tagtactgct tcaactaagt cctcaggttc tggaaaaatg tcatatacat 7140
   ctccaggtag acagatgagc caacagaacc ttaccaaaca aacaggttta tccaagaatg 7200
   ccagtagtat tccaagaagt gagtctgcct ccaaaggact aaatcagatg aataatggta 7260
   atggagccaa taaaaaggta gaactttcta gaatgtcttc aactaaatca agtggaagtg 7320
   aatctgatag atcagaaaga cctgtattag tacgccagtc aactttcatc aaagaagctc 7380
   caagcccaac cttaagaaga aaattggagg aatctgcttc atttgaatct ctttctccat 7440
   catctagacc agcttctccc actaggtccc aggcacaaac tccagtttta agtccttccc 7500
   ttcctgatat gtctctatcc acacattcgt ctgttcaggc tggtggatgg cgaaaactcc 7560
   cacctaatct cagtcccact atagagtata atgatggaag accagcaaag cgccatgata 7620
   ttgcacggtc tcattctgaa agtccttcta gacttccaat caataggtca ggaacctgga 7680
   aacgtgagca cagcaaacat tcatcatccc ttcctcgagt aagcacttgg agaagaactg 7740
   gaagttcatc ttcaattctt tctgcttcat cagaatccag tgaaaaagca aaaagtgagg 7800
   atgaaaaaca tgtgaactct atttcaggaa ccaaacaaag taaagaaaac caagtatccg 7860
   caaaaggaac atggagaaaa ataaaagaaa atgaattttc tcccacaaat agtacttctc 7920
   agaccgtttc ctcaggtgct acaaatggtg ctgaatcaaa gactctaatt tatcaaatgg 7980
   cacctgctgt ttctaaaaca gaggatgttt gggtgagaat tgaggactgt cccattaaca 8040
   atcctagatc tggaagatct cccacaggta atactccccc ggtgattgac agtgtttcag 8100
   aaaaggcaaa tccaaacatt aaagattcaa aagataatca ggcaaaacaa aatgtgggta 8160
   atggcagtgt tcccatgcgt accgtgggtt tggaaaatcg cctgaactcc tttattcagg 8220
   tggatgcccc tgaccaaaaa ggaactgaga taaaaccagg acaaaataat cctgtccctg 8280
   tatcagagac taatgaaagt tctatagtgg aacgtacccc attcagttct agcagctcaa 8340
   gcaaacacag ttcacctagt gggactgttg ctgccagagt gactcctttt aattacaacc 8400
   caagccctag gaaaagcagc gcagatagca cttcagctcg gccatctcag atcccaactc 8460
   cagtgaataa caacacaaag aagcgagatt ccaaaactga cagcacagaa tccagtggaa 8520
   cccaaagtcc taagcgccat tctgggtctt accttgtgac atctgtttaa aagagaggaa 8580
   gaatgaaact aagaaaattc tatgttaatt acaactgcta tatagacatt ttgtttcaaa 8640
   tgaaacttta aaagactgaa aaattttgta aataggtttg attcttgtta gagggttttt 8700
   gttctggaag ccatatttga tagtatactt tgtcttcact ggtcttattt tgggaggcac 8760
   tcttgatggt taggaaaaaa atagtaaagc caagtatgtt tgtacagtat gttttacatg 8820
   tatttaaagt agcatcccat cccaacttcc tttaattatt gcttgtctta aaataatgaa 8880
   cactacagat agaaaatatg atatattgct gttatcaatc atttctagat tataaactga 8940
   ctaaacttac atcagggaaa aattggtatt tatgcaaaaa aaaatgtttt tgtccttgtg 9000
   agtccatcta acatcataat taatcatgtg gctgtgaaat tcacagtaat atggttcccg 9060
   atgaacaagc tttacccagc ctgtttgctt tactgcatga atgaaactga tggttcaatt 9120
   tcagaagtaa tgattaacag ttatgtggtc acatgatgtg catagagata gctacagtgt 9180
   aataatttac actattttgt gctccaaaca aaacaaaaat ctgtgtaact gtaaaacatt 9240
   gaatgaaact attttacctg aactagattt tatctgaaag taggtagaat ttttgctatg 9300
   ctgtaatttg ttgtatattc tggtatttga ggtgagatgg ctgctctttt attaatgaga 9360
   catgaattgt gtctcaacag aaactaaatg aacatttcag aataaattat tgctgtatgt 9420
   aaactgttac tgaaattggt atttgtttga agggtcttgt ttcacatttg tattaataat 9480
   tgtttaaaat gcctctttta aaagcttata taaatttttt ncttcagctt ctatgcatta 9540
   agagtaaaat tcctcttact gtaataaaaa caattgaaga agactgttgc cacttaacca 9600
   ttccatgcgt tggcacttat ctattcctga aattctttta tgtgattagc tcatcttgat 9660
   ttttaacatt tttccactta aacttttttt tcttactcca ctggagctca gtaaaagtaa 9720
   attcatgtaa tagcaatgca agcagcctag cacagactaa gcattgagca taataggccc 9780
   acataatttc ctctttctta atattataga aattctgtac ttgaaattga ttcttagaca 9840
   ttgcagtctc ttcgaggctt tacagtgtaa actgtcttgc cccttcatct tcttgttgca 9900
   actgggtctg acatgaacac tttttatcac cctgtatgtt agggcaagat ctcagcagtg 9960
   aagtataatc agcactttgc catgctcaga aaattcaaat cacatggaac tttagaggta 10020
   gatttaatac gattaagata ttcagaagta tattttagaa tccctgcctg ttaaggaaac 10080
   tttatttgtg gtaggtacag ttctggggta catgttaagt gtccccttat acagtggagg 10140
   gaagtcttcc ttcctgaagg aaaataaact gacacttatt aactaagata atttacttaa 10200
   tatatcttcc ctgatttgtt ttaaaagatc agagggtgac tgatgataca tgcatacata 10260
   tttgttgaat aaatgaaaat ttatttttag tgataagatt catacactct gtatttgggg 10320
   agagaaaacc tttttaagca tggtggggca ctcagatagg agtgaataca cctacctggt 10380
   ggtcat 10386
<210> 61
   <211> 7273
   <212> DNA
   <213> Homo sapiens
<220>
   <221> misc_feature
   <223> PTGS2
   >gi|3282785|gb|AF044206.1| Homo sapiens cyclooxygenase (COX-2) gene, promoter and exon 1
<400> 61
<210> 62
   <211> 4465
   <212> DNA
   <213> Homo sapiens
<220>
   <221> misc_feature
   <223> >gi|4506264|ref|NM_000963.1| Homo sapiens prostaglandin-endoperoxide synthase 2 (prostaglandin G/H synthase and cyclooxygenase) (PTGS2), mRNA
<400> 62
   caattgtcat acgacttgca gtgagcgtca ggagcacgtc caggaactcc tcagcagcgc 60
   ctccttcagc tccacagcca gacgccctca gacagcaaag cctacccccg cgccgcgccc 120
   tgcccgccgc tcggatgctc gcccgcgccc tgctgctgtg cgcggtcctg gcgctcagcc 180
   atacagcaaa tccttgctgt tcccacccat gtcaaaaccg aggtgtatgt atgagtgtgg 240
   gatttgacca gtataagtgc gattgtaccc ggacaggatt ctatggagaa aactgctcaa 300
   caccggaatt tttgacaaga ataaaattat ttctgaaacc cactccaaac acagtgcact 360
   acatacttac ccacttcaag ggattttgga acgttgtgaa taacattccc ttccttcgaa 420
   atgcaattat gagttatgtc ttgacatcca gatcacattt gattgacagt ccaccaactt 480
   acaatgctga ctatggctac aaaagctggg aagccttctc taacctctcc tattatacta 540
   gagcccttcc tcctgtgcct gatgattgcc cgactccctt gggtgtcaaa ggtaaaaagc 600
   agcttcctga ttcaaatgag attgtggaaa aattgcttct aagaagaaag ttcatccctg 660
   atccccaggg ctcaaacatg atgtttgcat tctttgccca gcacttcacg catcagtttt 720
   tcaagacaga tcataagcga gggccagctt tcaccaacgg gctgggccat ggggtggact 780
   taaatcatat ttacggtgaa actctggcta gacagcgtaa actgcgcctt ttcaaggatg 840
   gaaaaatgaa atatcagata attgatggag agatgtatcc tcccacagtc aaagatactc 900
   aggcagagat gatctaccct cctcaagtcc ctgagcatct acggtttgct gtggggcagg 960
   aggtctttgg tctggtgcct ggtctgatga tgtatgccac aatctggctg cgggaacaca 1020
   acagagtatg cgatgtgctt aaacaggagc atcctgaatg gggtgatgag cagttgttcc 1080
   agacaagcag gctaatactg ataggagaga ctattaagat tgtgattgaa gattatgtgc 1140
   aacacttgag tggctatcac ttcaaactga aatttgaccc agaactactt ttcaacaaac 1200
   aattccagta ccaaaatcgt attgctgctg aatttaacac cctctatcac tggcatcccc 1260
   ttctgcctga cacctttcaa attcatgacc agaaatacaa ctatcaacag tttatctaca 1320
   acaactctat attgctggaa catggaatta cccagtttgt tgaatcattc accaggcaaa 1380
   ttgctggcag ggttgctggt ggtaggaatg ttccacccgc agtacagaaa gtatcacagg 1440
   cttccattga ccagagcagg cagatgaaat accagtcttt taatgagtac cgcaaacgct 1500
   ttatgctgaa gccctatgaa tcatttgaag aacttacagg agaaaaggaa atgtctgcag 1560
   agttggaagc actctatggt gacatcgatg ctgtggagct gtatcctgcc cttctggtag 1620
   aaaagcctcg gccagatgcc atctttggtg aaaccatggt agaagttgga gcaccattct 1680
   ccttgaaagg acttatgggt aatgttatat gttctcctgc ctactggaag ccaagcactt 1740
   ttggtggaga agtgggtttt caaatcatca acactgcctc aattcagtct ctcatctgca 1800
   ataacgtgaa gggctgtccc tttacttcat tcagtgttcc agatccagag ctcattaaaa 1860
   cagtcaccat caatgcaagt tcttcccgct ccggactaga tgatatcaat cccacagtac 1920
   tactaaaaga acgttcgact gaactgtaga agtctaatga tcatatttat ttatttatat 1980
   gaaccatgtc tattaattta attatttaat aatatttata ttaaactcct tatgttactt 2040
   aacatcttct gtaacagaag tcagtactcc tgttgcggag aaaggagtca tacttgtgaa 2100
   gacttttatg tcactactct aaagattttg ctgttgctgt taagtttgga aaacagtttt 2160
   tattctgttt tataaaccag agagaaatga gttttgacgt ctttttactt gaatttcaac 2220
   ttatattata agaacgaaag taaagatgtt tgaatactta aacactatca caagatggca 2280
   aaatgctgaa agtttttaca ctgtcgatgt ttccaatgca tcttccatga tgcattagaa 2340
   gtaactaatg tttgaaattt taaagtactt ttggttattt ttctgtcatc aaacaaaaac 2400
   aggtatcagt gcattattaa atgaatattt aaattagaca ttaccagtaa tttcatgtct 2460
   actttttaaa atcagcaatg aaacaataat ttgaaatttc taaattcata gggtagaatc 2520
   acctgtaaaa gcttgtttga tttcttaaag ttattaaact tgtacatata ccaaaaagaa 2580
   gctgtcttgg atttaaatct gtaaaatcag atgaaatttt actacaattg cttgttaaaa 2640
   tattttataa gtgatgttcc tttttcacca agagtataaa cctttttagt gtgactgtta 2700
   aaacttcctt ttaaatcaaa atgccaaatt tattaaggtg gtggagccac tgcagtgtta 2760
   tctcaaaata agaatatttt gttgagatat tccagaattt gtttatatgg ctggtaacat 2820
   gtaaaatcta tatcagcaaa agggtctacc tttaaaataa gcaataacaa agaagaaaac 2880
   caaattattg ttcaaattta ggtttaaact tttgaagcaa actttttttt atccttgtgc 2940
   actgcaggcc tggtactcag attttgctat gaggttaatg aagtaccaag ctgtgcttga 3000
   ataacgatat gttttctcag attttctgtt gtacagttta atttagcagt ccatatcaca 3060
   ttgcaaaagt agcaatgacc tcataaaata cctcttcaaa atgcttaaat tcatttcaca 3120
   cattaatttt atctcagtct tgaagccaat tcagtaggtg cattggaatc aagcctggct 3180
   acctgcatgc tgttcctttt cttttcttct tttagccatt ttgctaagag acacagtctt 3240
   ctcatcactt cgtttctcct atttgttttt actagtttta agatcagagt tcactttctt 3300
   tggactctgc ctatattttc ttacctgaac ttttgcaagt tttcaggtaa acctcagctc 3360
   aggactgcta tttagctcct cttaagaaga ttaaaagaga aaaaaaaagg cccttttaaa 3420
   aatagtatac acttatttta agtgaaaagc agagaatttt atttatagct aattttagct 3480
   atctgtaacc aagatggatg caaagaggct agtgcctcag agagaactgt acggggtttg 3540
   tgactggaaa aagttacgtt cccattctaa ttaatgccct ttcttattta aaaacaaaac 3600
   caaatgatat ctaagtagtt ctcagcaata ataataatga cgataatact tcttttccac 3660
   atctcattgt cactgacatt taatggtact gtatattact taatttattg aagattatta 3720
   tttatgtctt attaggacac tatggttata aactgtgttt aagcctacaa tcattgattt 3780
   ttttttgtta tgtcacaatc agtatatttt ctttggggtt acctctctga atattatgta 3840
   aacaatccaa agaaatgatt gtattaagat ttgtgaataa atttttagaa atctgattgg 3900
   catattgaga tatttaaggt tgaatgtttg tccttaggat aggcctatgt gctagcccac 3960
   aaagaatatt gtctcattag cctgaatgtg ccataagact gaccttttaa aatgttttga 4020
   gggatctgtg gatgcttcgt taatttgttc agccacaatt tattgagaaa atattctgtg 4080
   tcaagcactg tgggttttaa tatttttaaa tcaaacgctg attacagata atagtattta 4140
   tataaataat tgaaaaaaat tttcttttgg gaagagggag aaaatgaaat aaatatcatt 4200
   aaagataact caggagaatc ttctttacaa ttttacgttt agaatgttta aggttaagaa 4260
   agaaatagtc aatatgcttg tataaaacac tgttcactgt tttttttaaa aaaaaaactt 4320
   gatttgttat taacattgat ctgctgacaa aacctgggaa tttgggttgt gtatgcgaat 4380
   gtttcagtgc ctcagacaaa tgtgtattta acttatgtaa aagataagtc tggaaataaa 4440
   tgtctgttta tttttgtact attta 4465
<210> 63
   <211> 1336
   <212> DNA
   <213> Homo sapiens
<220>
   <221> misc_feature
   <223> 14-3-3-Sigma
   >gi|45238846|ref|NM-006142.3| Homo sapiens stratifin (SFN), mRNA
<400> 63
   gagagacaca gagtccggca ttggtcccag gcagcagtta gcccgccgcc cgcctgtgtg 60
   tccccagagc catggagaga gccagtctga tccagaaggc caagctggca gagcaggccg 120
   aacgctatga ggacatggca gccttcatga aaggcgccgt ggagaagggc gaggagctct 180
   cctgcgaaga gcgaaacctg ctctcagtag cctataagaa cgtggtgggc ggccagaggg 240
   ctgcctggag ggtgctgtcc agtattgagc agaaaagcaa cgaggagggc tcggaggaga 300
   aggggcccga ggtgcgtgag taccgggaga aggtggagac tgagctccag ggcgtgtgcg 360
   acaccgtgct gggcctgctg gacagccacc tcatcaagga ggccggggac gccgagagcc 420
   gggtcttcta cctgaagatg aagggtgact actaccgcta cctggccgag gtggccaccg 480
   gtgacgacaa gaagcgcatc attgactcag cccggtcagc ctaccaggag gccatggaca 540
   tcagcaagaa ggagatgccg cccaccaacc ccatccgcct gggcctggcc ctgaactttt 600
   ccgtcttcca ctacgagatc gccaacagcc ccgaggaggc catctctctg gccaagacca 660
   ctttcgacga ggccatggct gatctgcaca ccctcagcga ggactcctac aaagacagca 720
   ccctcatcat gcagctgctg cgagacaacc tgacactgtg gacggccgac aacgccgggg 780
   aagagggggg cgaggctccc caggagcccc agagctgagt gttgcccgcc accgccccgc 840
   cctgccccct ccagtccccc accctgccga gaggactagt atggggtggg aggccccacc 900
   cttctcccct aggcgctgtt cttgctccaa agggctccgt ggagagggac tggcagagct 960
   gaggccacct ggggctgggg atcccactct tcttgcagct gttgagcgca cctaaccact 1020
   ggtcatgccc ccacccctgc tctccgcacc cgcttcctcc cgaccccagg accaggctac 1080
   ttctcccctc ctcttgcctc cctcctgccc ctgctgcctc tgatcgtagg aattgaggag 1140
   tgtcccgcct tgtggctgag aactggacag tggcaggggc tggagatggg tgtgtgtgtg 1200
   tgtgtgtgtg tgtgtgtgtg tgtgcgcgcg cgccagtgca agaccgagat tgagggaaag 1260
   catgtctgct gggtgtgacc atgtttcctc tcaataaagt tcccctgtga cactcaaaaa 1320
   aaaaaaaaaa aaaaaa 1336
<210> 64
   <211> 1968
   <212> DNA
   <213> Homo sapiens
<220>
   <221> misc_feature
   <223> RASSF1A
   >gi|25777678|ref|NM_007182.4| Homo sapiens Ras association (RaIGDS/AF-6) domain family 1 (RASSF1), transcript variant A,mRNA
<400> 64
   tctcctcagc tccttcccgc cgcccagtct ggatcctggg ggaggcgctg aagtcggggc 60
   ccgccctgtg gccccgcccg gcccgcgctt gctagcgccc aaagccagcg aagcacgggc 120
   ccaaccgggc catgtcgggg gagcctgagc tcattgagct gcgggagctg gcacccgctg 180
   ggcgcgctgg gaagggccgc acccggctgg agcgtgccaa cgcgctgcgc atcgcgcggg 240
   gcaccgcgtg caaccccaca cggcagctgg tccctggccg tggccaccgc ttccagcccg 300
   cggggcccgc cacgcacacg tggtgcgacc tctgtggcga cttcatctgg ggcgtcgtgc 360
   gcaaaggcct gcagtgcgcg cattgcaagt tcacctgcca ctaccgctgc cgcgcgctcg 420
   tctgcctgga ctgttgcggg ccccgggacc tgggctggga acccgcggtg gagcgggaca 480
   cgaacgtgga cgagcctgtg gagtgggaga cacctgacct ttctcaagct gagattgagc 540
   agaagatcaa ggagtacaat gcccagatca acagcaacct cttcatgagc ttgaacaagg 600
   acggttctta cacaggcttc atcaaggttc agctgaagct ggtgcgccct gtctctgtgc 660
   cctccagcaa gaagccaccc tccttgcagg atgcccggcg gggcccagga cggggcacaa 720
   gtgtcaggcg ccgcacttcc ttttacctgc ccaaggatgc tgtcaagcac ctgcatgtgc 780
   tgtcacgcac aagggcacgt gaagtcattg aggccctgct gcgaaagttc ttggtggtgg 840
   atgacccccg caagtttgca ctctttgagc gcgctgagcg tcacggccaa gtgtacttgc 900
   ggaagctgtt ggatgatgag cagcccctgc ggctgcggct cctggcaggg cccagtgaca 960
   aggccctgag ctttgtcctg aaggaaaatg actctgggga ggtgaactgg gacgccttca 1020
   gcatgcctga actacataac ttcctacgta tcctgcagcg ggaggaggag gagcacctcc 1080
   gccagatcct gcagaagtac tcctattgcc gccagaagat ccaagaggcc ctgcacgcct 1140
   gccccctggg gtgacctctt gtacccccag gtggaaggca gacagcaggc agcgccaagt 1200
   gcgtgccgtg tgagtgtgac agggccagtg gggcctgtgg aatgagtgtg catggaggcc 1260
   ctcctgtgct gggggaatga gcccagagaa cagcgaagta gcttgctccc tgtgtccacc 1320
   tgtgggtgta gccaggtatg gctctgcacc cctctgccct cattactggg ccttagtggg 1380
   ccagggctgc cctgagaagc tgctccaggc ctgcagcagg agtggtgcag acagaagtct 1440
   cctcaatttt tgtctcagaa gtgaaaatct tggagaccct gcaaacagaa cagggtcatg 1500
   tttgcagggg tgacggccct catctatgag gaaaggtttt ggatcttgaa tgtggtctca 1560
   ggatatcctt atcagagcta agggtgggtg ctcagaataa ggcaggcatt gaggaagagt 1620
   cttggtttct ctctacagtg ccaactcctc acacaccctg aggtcaggga gtgctggctc 1680
   acagtacagc atgtgcctta atgcttcata tgaggaggat gtccctgggc cagggtctgt 1740
   gtgaatgtgg gcactggccc aggttcatac cttatttgct aatcaaagcc agggtctctc 1800
   cctcaggtgt tttttatgaa gtgcgtgaat gtatgtaatg tgtggtggcc tcagctgaat 1860
   gcctcctgtg gggaaagggg ttggggtgac agtcatcatc agggcctggg gcctgagaga 1920
   attggctcaa taaagatttc aagatcctca aaaaaaaaaa aaaaaaaa 1968
<210> 65
   <211> 17
   <212> DNA
   <213> Artificial
<220>
   <223> GSTP1 primer
<400> 65
   agttgcgcgg cgatttc 17
<210> 66
   <211> 23
   <212> DNA
   <213> Artificial
<220>
   <223> Probe (5'FAM/3'TAMRA) GSTP1
<400> 66
   cggtcgacgt tcggggtgta gcg 23